# EUROPEAN PATENT SPECIFICATION

(11) **EP 3 263 180 B1**
(45) Date of publication and mention of the grant of the patent: **08.04.2020**
(21) Application number: 16177338.7
(22) Date of filing: 30.06.2016
(51) Int. Cl.: A61Q 5/06, A61K 8/81, A61K 8/85

(54) **METHOD FOR COLORING HAIR FIBERS INTO A FIRST AND A SECOND PATTERNS**
VERFAHREN ZUM FÄRBEN VON HAARFASERN IN EINEM ERSTEN UND IN EINEM ZWEITEN MUSTER
PROCÉDÉ DE COLORATION DE FIBRES DE POILS EN UN PREMIER ET UN SECOND MOTIFS

(43) Date of publication of application: 03.01.2018
(73) Proprietor: Noxell Corporation, Hunt Valley, Maryland 21030-2098 (US)
(72) Inventor: KOCH, Malte, 65824 Schwalbach am Taunus (DE); FUCHS, Sybille, 65824 Schwalbach am Taunus (DE); BECKER, Martina, 65824 Schwalbach am Taunus (DE); FINDEIS, Bernd, 65824 Schwalbach am Taunus (DE)
(74) Representative: Bandpay & Greuter

(56) References cited:
- EP-A2- 2 574 331
- FR-A1- 2 992 558
- US-A- 4 074 964

## Description

### FIELD OF THE INVENTION

A method for coloring hair fibers into a first and a second patterns, preferably human hair fibers, wherein the hair fibers have one or more initial color shades, is provided and comprises the following steps in that order of providing an adhesive composition, wherein the adhesive composition comprises, in a cosmetically acceptable carrier, a film-forming polymer, providing a hair coloring composition for forming a final color shade, which differ from the one or more initial color shades. The application of the adhesive composition to a first plurality of hair fiber portions to form a first pattern and then the application of the hair coloring composition to a second plurality of hair fiber portions including the first plurality of hair fiber portions comprising the first pattern, allows producing accurate colored patterns.

Also, a kit for coloring hair fibers into a first and a second patterns is provided and comprises an adhesive composition, a dye composition and optionally a developer composition separately packaged in different containers or packaged in a same container in different compartments; and an instructional material.

### BACKGROUND OF THE INVENTION

Hair coloring or dyeing involves the application of one or more hair dyes onto hair which results in the coloration of hair fibers. The total head of hair color may be changed subtly or dramatically, the root growth colored to match the remaining head of hair, effects introduced such as glitter, hair strand effects or other sectional effects, or the same color "freshened up" to combat fade and/or wash-out.

It is known to dye keratinous fibres, and in particular human hair comprising un-pigmented hair, with dyeing compositions containing oxidation dye precursors. Oxidation dye precursors, or oxidation bases, are colourless or slightly coloured compounds which, when mixed with oxidizing products at the time of use, are able, by a process of oxidative condensation, to give rise to coloured compounds and dyes. It is also known that it is possible to vary the shades obtained with these oxidation bases by combining them with couplers or dye modifiers.

While it is relatively easy to change the color of the hair, as a whole, by dyeing to make it darker, or by bleaching to lighten it, the result of only dyeing or only bleaching may be disappointing. The hair so treated tends to have an uninteresting uniform color. It is customary to follow dyeing or bleaching operation with another step, in which selected strands of hair are further treated so that their color will change and so that these selected strands will stand out against the background provided by the remainder of the hair. Similarly, selected strands of hair of a person's natural hair can be treated. Treatment of selected strands of hair to change the color is called streaking for instance, if the selected strands are lightened, or reverse streaking if they are darkened.

The task of coloring selected strands of hair is difficult for a hairdresser, since many strands of hair all over the head must be individually treated with appropriate treating material, while guarding the general mass of hair from contact with the treating material, and it is arduous for the client because of the length of time involved.

Up to now, substantially two methods have been known for generating streaks. In the first method, a thin elastic cap in the form of a bathing cap is put on the person's head, whose hair is to be streaked, into which cap holes are pricked with a needle in the form of a crochet hook and fine strands are pulled out towards the outside. On these strands hair colorants are applied. In a similar method, strands of the hair to be dyed are isolated by a comb, a sheet of aluminum foil is inserted below the strands, the hair colorant is applied to the strands by a brush, and subsequently the aluminum foils are folded around the strands and closed.

Alternatively, a plurality of colored streaks can be created by a method comprising painstakingly applying a plurality of sticky tapes to the hair to partially block coloring in the desired areas. This method is feasible temporary for shows and photo-shoots, however not in a commercially viable consumer relevant proposition. Both methods are very laborious and time-consuming.

In an alternative method, each strand to be dyed is treated with an applicator brush, on which the hair colorant is applied, by pulling the soaked applicator brush through the isolated strand starting from the root of the hair. In this way streaks are generated which have sharply marked edges, what gives an unnatural impression.

The above technologies are not suitable for providing very accurate and repetitive patterns on hair, especially when the patterns have relatively small or micro-sizes.

A method allowing the dyeing of hair with at least two different tones, with satisfactory color separation has been recently developed; see for Example European Patent Application EP 2 574 331 A2.

There is still a need for a hairdresser to develop a new high definition coloring method. Therefore, there is an interest in providing an improved method for creating precise color patterns on hair without any color fading. In particular, there is a need to develop an improved method for creating regular and/or repetitive color patterns on hair with well-defined contours that blend together when hair moves.

### SUMMARY OF THE INVENTION

A method for coloring hair fibers into a first and a second patterns, preferably human hair fibers, wherein the hair fibers have one or more initial color shades, is provided and comprises the following steps in that order:
(a) providing an adhesive composition, wherein the adhesive composition comprises, in a cosmetically acceptable carrier, a film-forming polymer in a total amount of film-forming polymer ranging from 10% to 75%, preferably from 15% to 65%, more preferably from 20% to 60%, even more preferably from 25% to 50% by total weight of the adhesive composition;
   wherein the film forming polymer comprises one or more polymers as defined in the appended claims;
   wherein the film-forming polymer is water-soluble or water-dispersible at 25°C;
   wherein the film-forming polymer has a glass transition temperature from -20°C to 100°C, preferably from -20°C to 70°C, more preferably from 0°C to 65°C;
   wherein the adhesive composition has a viscosity of from 100 centipoises to 75 000 centipoises, preferably from 400 centipoises to 3 000 centipoises according to the Viscosity Test Method;
(b) providing a hair coloring composition for forming a final color shade, which differ from the one or more initial color shades;
(c) applying the adhesive composition to a first plurality of hair fiber portions to form a first pattern, wherein the first plurality of hair fiber portions are covered and immobilized by the first pattern;
(d) drying the first plurality of hair fiber portions;
(e) applying the hair coloring composition to a second plurality of hair fiber portions including the first plurality of hair fiber portions comprising the first pattern, preferably to all the hair fibers;
(f) rinsing the adhesive composition and the hair coloring composition out of the hair fibers, and drying the hair fibers, to form a second pattern, wherein the second pattern comprises the second plurality of hair fiber portions without the first pattern, such that the hair fibers comprises the first pattern which has the one or more initial color shades and the second pattern which has the final color shade.

A kit for coloring hair fibers into a first and a second patterns, preferably human hair fibers, is provided and comprises:
(a) an adhesive composition,
   wherein the adhesive composition comprises, in a cosmetically acceptable carrier, a film-forming polymer in a total amount of film-forming polymer ranging from 10% to 75%, preferably from 15% to 65%, more preferably from 20% to 60%, even more preferably from 25% to 50% by total weight of the adhesive composition;
   wherein the film-forming polymer is water-soluble or water-dispersible at 25°C;
      wherein the film-forming polymer has a glass transition temperature from -20°C to 100°C, preferably from -20°C to 70°C, more preferably from 0°C to 65°C;
   wherein the adhesive composition has a viscosity of from 0,1 Pa.s to 75 Pa.s, preferably from 0,4 Pa.s to 3 Pa.s according to the Viscosity Test Method; and
   wherein the film-forming polymer comprises one or more polymers as defined in the appended claims;
(b) a dye composition, preferably comprising one or more oxidative dye precursors or consisting essentially of one or more direct dyes;
(c) optionally a developer composition comprising one or more oxidizing agents;
   wherein the adhesive composition, the dye composition (b) and optionally the developer composition (c) are separately packaged in different containers or packaged in a same container in different compartments;
(d) an instructional material for preparation, for use, or both preparation and use, of the adhesive composition, the dye composition and optionally the developer composition; and
(e) optionally an application tool for the adhesive composition.
The adhesive composition may be supplied as a single-use disposable dose.

### BRIEF DESCRIPTION OF THE DRAWINGS

Fig. 1 is a schematic side view, showing the hair fibers comprising a first pattern which has the initial color shade and a second pattern which has a final color shade;
Fig. 2 is a schematic view of a first plurality of hair fiber portions on which an adhesive composition is applied by the means of an application tool comprising a relatively slim nozzle applicator;
Fig. 3 is another schematic side view, showing the hair fibers comprising a first pattern which has an initial color shade and a second pattern which has a final color shade;
Fig. 4A illustrates a schematic view of the hair fibers after application of an adhesive composition to a first plurality of hair fiber portions to form a first pattern;
Fig. 4B illustrates a schematic view of the hair fibers of Fig. 4A after application of a hair coloring composition to all the hair fibers; and
Fig. 4C illustrates a schematic final view of the hair fibers as Fig. 3, after rinsing the adhesive composition and the hair coloring composition out of the hair fibers of Fig. 4B, and drying the hair fibers, to form a second pattern, wherein the second pattern comprises all the hair fibers without the first pattern, such that the hair fibers comprises the first pattern which has the initial color shade and the second pattern which has the final color shade.

### DETAILED DESCRIPTION OF THE INVENTION

### Definitions of terms

In this document, including in all embodiments of all aspects of the present invention, the following definitions apply unless specifically stated otherwise.

All percentages are by weight (w/w) of the respective composition, unless otherwise specified. All ratios or percentages are weight ratios or weight percentages unless specifically stated otherwise."% wt." means percentage by weight. References to "parts" e.g. a mixture of 1 part X and 3 parts Y, is a ratio by weight. When more than one composition are used during a treatment, the total weight to be considered is the total weight of all the compositions applied on the hair simultaneously (i.e. the weight found "on head"), unless otherwise specified.

"QSP" means sufficient quantity for 100% or for 100g. "+/-" indicates the standard deviation. All ranges are inclusive and combinable. The number of significant digits conveys neither a limitation on the indicated amount nor on the accuracy of the measurement. All numerical amounts are understood to be modified by the word "about".

All measurements are understood to be made at 20°C and at ambient conditions, where "ambient conditions" means at 1 atmosphere (atm) of pressure and at 65% relative humidity, unless otherwise stated. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International.

Herein "min" means "minute" or "minutes". Herein "mol" means mole. Herein "g" following a number means "gram" or "grams". "Ex." means "example". All amounts as they pertain to listed ingredients are based on the active level ("solids") and do not include carriers or by-products that may be included in commercially available materials.

Herein, "comprising" means that other steps and other ingredients can be in addition. "Comprising" encompasses the terms "consisting of' and "consisting essentially of". The compositions, methods, uses, and kits of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated.

Where amount ranges are given, these are to be understood as being the total amount of said ingredient in the composition, or where more than one species fall within the scope of the ingredient definition, the total amount of all ingredients fitting that definition, in the composition.

For example, if the composition comprises from 1% to 5% fatty alcohol, then a composition comprising 2% stearyl alcohol and 1% cetyl alcohol and no other fatty alcohol, would fall within this scope.

The amount of each particular ingredient or mixtures thereof described hereinafter can account for up to 100% (or 100%) of the total amount of the ingredient(s) in the composition.

The term "substantially free of' as used herein means less than 1%, less than 0.8%, less than 0.5%, less than 0.3%, or less than an immaterial amount of by total weight of the composition.

The term "hair" as used herein means mammalian hair including scalp hair, facial hair and body hair, more preferably hair on the human head and scalp. Hair comprises hair fibers. "Hair shaft" means an individual hair strand and may be used interchangeably with the term "hair." As used herein the term "hair" to be treated may be "living" i.e. on a living body or may be "non-living" i.e. in a wig, hairpiece or other aggregation of non-living keratinous fibers. Mammalian, preferably human hair is preferred.

The term "hair fiber portion" as used herein means from any part of a hair fiber across a length of the hair fiber to the whole length of the hair fiber, and/or the root of the hair fiber, and/or the tip of the hair fiber.

The term "film-forming polymer" as used herein means a polymer which is in a position to deposit a polymer film on the hair.

The term "copolymer" as used herein refers to a polymer derived from two or more polymerizable monomers. When used in generic terms, the term "copolymer" is also inclusive of more than two distinct monomers, for example, ter-polymers.

The term "cosmetically acceptable" as used herein means that the compositions, or components described are suitable for use in contact with human keratinous tissue without undue toxicity, incompatibility, instability, allergic response, and the like. All compositions described herein which have the purpose of being directly applied to keratinous tissue are limited to those being cosmetically acceptable.

The term "separately packaged" as used herein means any form of packaging that prevents a first composition from coming into physical contact, or admixing, with a second or a third composition. "Separately packaged" may mean that the individual first, second and third compositions are packaged in separate containers, or alternatively in a single container partitioned such that the first, second and third compositions are not in physical contact.

The term "kit" as used herein means a packaging unit comprising a plurality of components i.e. a kit of parts. An example of a kit is, for example, a first composition, a separately packaged second composition and a separately packaged third composition. Another kit may comprise application instructions comprising a method and a first, second and third compositions.

### Method for coloring hair fibers into a first and a second patterns

A method for coloring hair fibers into a first and a second patterns, preferably human hair fibers, wherein the hair fibers have one or more initial color shades, is provided. The method comprises the following steps in that order of providing an adhesive composition, wherein the adhesive composition comprises, in a cosmetically acceptable carrier, a film-forming polymer; providing a hair coloring composition providing a hair coloring composition for forming a final color shade, which differ from the one or more initial color shades.

The first part of the method deals with applying the adhesive composition to a first plurality of hair fiber portions to form a first pattern; and drying the first plurality of hair fiber portions.

The second part of the method concerns applying the hair coloring composition to a second plurality of hair fiber portions including the first plurality of hair fiber portions comprising the first pattern; rinsing the adhesive composition and the hair coloring composition out of the hair fibers, and drying the hair fibers, to form a second pattern.

The second pattern comprises the second plurality of hair fiber portions without the first pattern, such that the hair fibers comprises the first pattern which has the one or more initial color shades and the second pattern which has the final color shade.

The present inventors have found that it is possible to provide accurate color patterns on hair without any color fading and any use of physical barriers like aluminum foils. In particular, the method allows creating regular and/or repetitive color patterns on hair with well-defined contours that blend together when hair moves.

Indeed, when applying the adhesive composition to a first plurality of hair fiber portions, a first pattern is formed. The first plurality of hair fiber portions are covered and immobilized by the first pattern. Hence, the first plurality of hair fiber portions are covered and immobilized by the adhesive composition forming the first pattern. As the first plurality of hair fiber portions are covered by the adhesive composition at the first pattern, the adhesive composition acts a relatively thin barrier protection against the coloring components of the hair coloring composition such as oxidative dyes, direct dyes or against the bleaching oxidizing agents.

Hence, when the hair coloring composition is applied to the second plurality of hair fiber portions, wherein the second plurality of hair fiber portions include the first pattern, or preferably to all the hair fibers, the coloring ingredients of the hair color composition or the bleaching oxidizing agents cannot go through the adhesive composition, such that the first pattern cannot be colored or bleached. Only the rest of the hair fibers, i.e. the second plurality of hair fiber portions without the first pattern, can be colored or bleached.

As a consequence, the hair fibers comprises the first pattern which keeps the one or more initial color shades and the second pattern which has the final color shade.

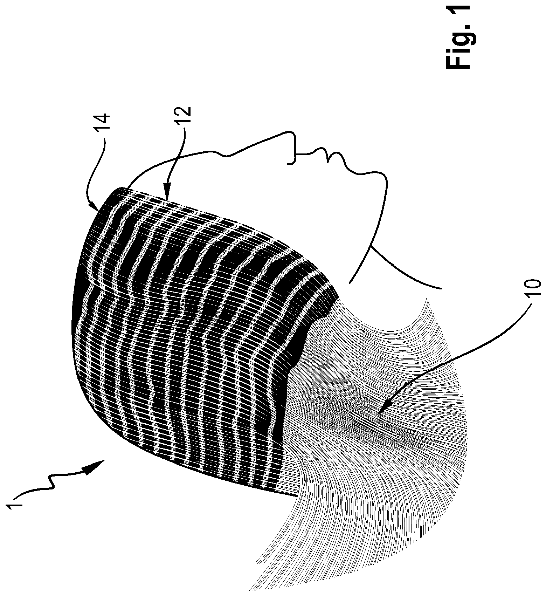

Fig. 1 is a schematic side view, showing the hair fibers comprising a first pattern which has the initial color shade and a second pattern which has a final color shade. The hair 1 has an initial color shade 10. After the hair being treated according to the method of the present invention, the hair comprises a first pattern 12 which has the initial color shade; and a second pattern 14 which has the final color shade resulting from the application of the adhesive composition and then of the hair coloring composition.

With this method, there is no need to use any barriers such as foil, plastic, film, cotton wool, padding material, caps, etc, in order to define the contours of the first and the second patterns with accuracy. Now, the proposed method even allows providing to the hair fibers regular and/or repetitive color patterns with well-defined contours and with a relatively small size.

The first plurality of hair fiber portions still having the one or more initial color shades may be further colored or bleached in a subsequent step. It is possible to control the hair coloration of the hair fibers regiospecifically.

Also, the adhesive composition can help to weld together the first plurality of hair fibers. When the consumer moves, the hair is immobilized by the first pattern, which prevents the risk of fading of any hair color composition applied to the second plurality of hair fiber portion to another portion of the hair fibers which differ from the first and the second plurality of hair fiber portions. The another portion of the hair fibers may be still virgin of any hair color treatment, or may have already received a hair color treatment.

The features of the method and the associated benefits will be described in more detailed below.

### Provision of an adhesive composition

The method for coloring hair fibers into a first and a second patterns, preferably human hair fibers, wherein the hair fibers have one or more initial color shades, is provided. The method comprises the step of providing an adhesive composition. The adhesive composition comprises, in a cosmetically acceptable carrier, a film-forming polymer.

The adhesive composition comprises a total amount of film-forming polymer ranging from 10% to 75%, preferably from 15% to 65%, more preferably from 20% to 60%, even more preferably from 25% to 50% by total weight of the adhesive composition.

The adhesive composition has a viscosity of from 0,1 Pa.s to 75 Pa.s, preferably from 0,15 Pa.s to 50 Pa.s, more preferably from 0,2 Pa.s to 25 Pa.s, still preferably from 0,3 Pa.s to 10 Pa.s, more preferably from 0,4 Pa.s to 3 Pa.s according to the Viscosity Test Method. Indeed, the adhesive composition having the viscosity as stated herein before can be non-runny in order to avoid dropping from opened and inverted products containers and from the fingers or any application tools used to apply the adhesive composition to the hair fibers. The skilled person will know how to choose the sufficient viscosity by increasing the amount of the film-forming polymer in the adhesive composition.

Hence, the adhesive composition comprises a total amount of film-forming polymer ranging from 10% to 75%, by total weight of the adhesive composition and the adhesive composition has a viscosity of from 100 centipoises to 75 000 centipoises according to the Viscosity Test Method.

The adhesive composition may preferably comprise a total amount of film-forming polymer ranging from 20% to 60% by total weight of the adhesive composition, and the adhesive composition may have a viscosity of from 0,4 Pa.s to 3 Pa.s according to the Viscosity Test Method.

Hence, it is mostly preferred to provide an adhesive composition with a relatively high amount of film-forming polymer relative to the total weight of the adhesive composition. In that case, the adhesive composition is more viscous and less subjected to drop. Its application to the first plurality of hair fiber portions is then facilitated and accurate, in particular for obtaining a relatively micro-sized first pattern.

The film-forming polymer is water-soluble or water-dispersible at 25°C. The term "water-soluble or water-dispersible" means that the film-forming polymer which, when introduced into an aqueous phase at 25°C, to a mass concentration equal to 1%, make it possible to obtain a macroscopically homogeneous and transparent solution, i.e. a solution that has a maximum light transmittance value, at a wavelength equal to 500 nm, through a sample 1 cm thick, of at least 60% and preferably of at least 70%.

The film-forming polymer has a glass transition temperature (Tg) from -20°C to 100°C; preferably from -20°C to 70°C, more preferably from 0°C to 65°C, even more preferably from 0°C to 35°C. The glass transition temperature can be measured by differential scanning calorimetry (DSC) according to ASTM D3418-97.

Film-forming polymers may comprise one or more polymers, preferably polyesters or acrylic copolymers, selected from block, graft, branched and cross-linked copolymers derived from two or more monomers selected from the group consisting of (meth)acrylic acid, crotonic acid, itaconic acid, citraconic acid, aconitic acid, (C₁-C₄-alkyl)-esterof(meth)acrylic acid, (C₁-C₄-hydroxyalkyl)-ester of (meth)acrylic acid, (meth)acrylamide, N-(C₁-C₄-alkyl)(meth)acrylamide, maleic acid, fumaric acid, aryl-dicarboxylic acid, aryl-acid anhydride, (C₁-C₄-alkyl)-ester of aryl-dicarboxylic acid, aryl-sulfonated dicarboxylic acid, aryl-sulfonated acid anhydride, (C₁-C₄-alkyl)-ester of aryl-sulfonated dicarboxylic acid, (C₁-C₄-alkyl)-ester of aryl-sulfonated dicarboxylic acid, and mixtures thereof. (C₁-C₄-alkyl) may be either methyl, or ethyl, or propyl, or isopropyl, or n-butyl, or sec-butyl, or tertio-butyl. (C₁-C₄-hydroxyalkyl) may be hydroxymethyl, or hydroxyethyl, or hydroxypropyl, or hydroxybutyl.

Film-forming polymers may comprise one or more polyesters selected from copolymers derived from two or more monomers selected from the group consisting of isophthalic acid, terephthalic acid, sulfonated-isophthalic acid, sulfonated-terephthalic acid, (C₁-C₄-alkyl)-ester of sulfonated-isophthalic acid, (C₁-C₄-alkyl)-ester of sulfonated-terephthalic acid, and mixtures thereof; or one or more acrylic copolymers derived from two or more monomers selected from the group consisting of (meth)acrylic acid, (C₁-C₄-alkyl)-ester of (meth)acrylic acid, (C₁-C₄-hydroxyalkyl)-ester of (meth)acrylic acid, and mixtures thereof.

Film-forming polymers may comprise one or more polyesters which are aryl-sulfonated polyesters selected from copolymers derived from two or more monomers selected from the group consisting of isophthalic acid, terephthalic acid, 5-sulfonated-isophthalic acid, 5-sulfonated-terephthalic acid, (C₁-C₄-alkyl)-ester of 5-sulfonated-isophthalic acid, (C₁-C₄-alkyl)-ester of 5-sulfonated-terephthalic acid, and mixtures thereof; or one or more acrylic copolymers derived from two or more monomers selected from the group consisting of (meth)acrylic acid, (C₁-C₄-alkyl)-ester of (meth)acrylic acid, (C₁-C₄-hydroxyalky))-ester of (meth)acrylic acid, and mixtures thereof.

Film-forming polymers may comprise one or more acrylic copolymers derived from two or more monomers selected from the group consisting of (meth)acrylic acid, (C₁-C₄-alkyl)-ester of (meth)acrylic acid, (C₁-C₄-hydroxyalkyl)-ester of (meth)acrylic acid, and mixtures thereof.

The polyesters, preferably the aryl-sulfonated polyesters and the acrylic copolymers have both the common property to form a relatively thin film deposit on the first plurality of hair fiber portions. The relatively thin film deposit on the first plurality of hair fiber portions can help to block the penetration of the coloring ingredients of the hair coloring composition or the bleaching oxidizing agents, and thus acts as a temporary protecting barrier for the first plurality of hair fiber portions.

The aryl-sulfonated polyesters and the acrylic copolymers, according to the present invention and as defined in the appended claims, are detailed below.

### Aryl-sulfonated polyesters

Film-forming polymers may be aryl-sulfonated polyesters derived from two or more monomers selected from the group consisting of aryl-dicarboxylic acid, aryl-acid anhydride, (C₁-C₄-alkyl)-ester of aryl-dicarboxylic acid, aryl-sulfonated dicarboxylic acid, aryl-sulfonated acid anhydride, (C₁-C₄-alkyl)-ester of aryl-sulfonated dicarboxylic acid, and mixtures thereof. (C₁-C₄-alkyl) may be either methyl, or ethyl, or propyl, or isopropyl, or n-butyl, or sec-butyl, or tertio-butyl.

Film-forming polymers may comprise one or more polyesters selected from copolymers derived from two or more monomers selected from the group consisting of isophthalic acid, terephthalic acid, sulfonated-isophthalic acid, sulfonated-terephthalic acid, (C₁-C₄-alkyl)-ester of sulfonated-isophthalic acid, (C₁-C₄-alkyl)-ester of sulfonated-terephthalic acid, and mixtures thereof.

Film-forming polymers may comprise one or more polyesters selected from copolymers derived from two or more monomers selected from the group consisting of isophthalic acid, terephthalic acid, 5-sulfonated-isophthalic acid, 5-sulfonated-terephthalic acid, (C₁-C₄-alkyl)-ester of 5-sulfonated-isophthalic acid, (C₁-C₄-alkyl)-ester of 5-sulfonated-terephthalic acid, and mixtures thereof.

The term "aryl-sulfonated polyester", according to the present invention, means copolyesters obtained by polycondensation of at least one dicarboxylic acid or of an ester thereof, of at least one diol and of at least one sulfoaryldicarboxylic difunctional compound substituted on the aromatic nucleus with a sulfonated group -SO₃M in which M represents a hydrogen atom, an ammonium ion, substituted ammonium or a metal ion such as Na⁺, Li⁺ or K⁺.

The aryl-sulfonated polyesters may generally have a weight-average molecular mass of between 1 000 Da and 60 000 Da, preferably from 5 000 Da to 40 000 Da, more preferably from 20 000 Da to 30 000 Da as determined by gel permeation chromatography (or GPC).

The glass transition temperature (Tg) of these aryl-sulfonated polyesters is within the range from -20°C to 100°C, preferably within the range from 50°C to 100°C and preferably from 50°C to 70°C.

The aryl-sulfonated polyesters are described in greater detail in the following patent applications U.S. Pat. No. 3,734,874; U.S. Pat. No. 3,779,993; U.S. Pat. No. 4,119,680; U.S. Pat. No. 4,300,580; U.S. Pat. No. 4,973,656; U.S. Pat. No. 5,660,816; U.S. Pat. No. 5,662,893; and U.S. Pat. No. 5,674,479.

aryl-sulfonated polyester may be preferably a polycondensate of at least one dicarboxylic acid or of an ester thereof, of at least one diol and of at least one 5-sulfoaryldicarboxylic difunctional compound substituted on the aromatic nucleus with a sulfonated group - SO₃M in which M represents a hydrogen atom, an ammonium ion, substituted ammonium or a metal ion such as Na⁺, Li⁺ or K⁺.

The aryl-sulfonated polyester, according to the present invention, is obtained from isophthalic acid, from the sodium salt of sulfoisophthalic acid, from diethylene glycol and from 1,4-cyclohexane-methanol.

The aryl-sulfonated polyester preferably used in the invention may comprise at least units derived from isophthalic acid, from a salt of 5-sulfoaryldicarboxylic acid and from diethylene glycol, and more particularly, the aryl-sulfonated polyester used in the invention are obtained from isophthalic acid, from the sodium salt of 5-sulfoisophthalic acid, from diethylene glycol and from 1,4-cyclohexanemethanol.

The aryl-sulfonated polyesters may be prepared by melt-phase polymerization of glycols and aromatic diacids. Typical monomers used to produce the aryl-sulfonated polyesters are isophthalic acid or terephthalic acid, 5-sodium (or 5-potassium, or 5-lithium)-sulfoisophthalic acid, 5-sodium (or 5-potassium, or 5-lithium)-sulfoterephthalic acid, 1,4-cyclohexanedimethanol, and diethylene glycol.

Examples of aryl-sulfonated polyester that may especially be mentioned include those known under the INCI name Polyester-5 and sold under the trade name "Eastman AQ™ polymer" (by the company Eastman Chemical). Eastman AQ™ 38S, 48 Ultra, or 55S are non-limited examples of aryl-sulfonated polyesters that can be used in the adhesive composition of the present invention. They contain the four following monomers isophthalic acid, 5-sodium-sulfoisophthalic acid, 1,4-cyclohexanedimethanol, and diethylene glycol, but in different ratios.

As described hereinafter, an Example I1 of the adhesive composition comprising Eastman AQ™ 38S polymer as the film-forming polymer has been prepared and used according to the method of the present invention.

Also, an Example I2 of the adhesive composition comprising Eastman AQ™ 2350 polymer as the film-forming polymer has been prepared and used according to the method of the present invention. Eastman AQ™ 2350 polymer has a glass transition temperature of 10°C.

Both Eastman AQ™ 38S polymer and Eastman AQ™ 2350 polymer contain isophthalic acid, 5-sodiosulfoisophthalic acid, 1,4-cyclohexanedimethanol, and diethylene glycol, but in different ratios.

The adhesive composition may comprise a total amount of aryl-sulfonated polyester ranging from 10% to 75%, preferably from 15% to 65%, more preferably from 20% to 60%, even more preferably from 25% to 50% by total weight of the adhesive composition.

Unlike solution polymers whose chains entangle at low concentration, aryl-sulfonated polyester dispersions have a relatively low viscosity. The viscosity rises with increasing concentration.

The adhesive composition has a viscosity of from 0,1 Pa.s to 75 Pa.s, preferably from 0,4 Pa.s to 3 Pa.s according to the Viscosity Test Method.

The adhesive composition may comprise a total amount of aryl-sulfonated polyester ranging from 10% to 75%, by total weight of the adhesive composition and the adhesive composition may have a viscosity of from 0,1 Pa.s to 75 Pa.s according to the Viscosity Test Method.

The adhesive composition may preferably comprise a total amount of aryl-sulfonated polyester ranging from 20% to 60% by total weight of the adhesive composition, and the adhesive composition may have a viscosity of from 0,4 Pa.s to 3 Pa.s according to the Viscosity Test Method.

Aryl-sulfonated polyesters in aqueous dispersions exhibit good chemical stability at a pH ranging from 5 to 8. Hence, the adhesive composition may have a pH from 5 to 8, preferably from 6 to 7.5.

### Acrylic copolymers

Film-forming polymers may be acrylic copolymers. Film-forming polymer of the adhesive composition is a crosslinked acrylic copolymer which is obtained by polymerization from a monomer composition comprising three different monomer components, a1), b1), c1), and an optional fourth monomer component d).

The first monomer component a1) may be selected from one or more monoethylenically unsaturated monomers containing at least one carboxylic acid group.

The first monomer component a1) may be selected from the group consisting of (meth)acrylic acid, itaconic acid, citraconic acid, maleic acid, fumaric acid, crotonic acid, aconitic acid, and mixtures thereof. According to the present invention, the first monomer component a1) is (meth)acrylic acid.

The amount of the at least one carboxylic acid group containing monomer set forth under the first monomer component a1) may range from 10% to 80% by weight, preferably from 20% to 70% by weight, more preferably from 35% to 65% by weight, based upon the total weight of the monomers in the polymerizable monomer composition.

The second monomer component b1) may be selected from at least one monoethylenically unsaturated monomer containing at least one linear or branched C₁ to C₄ alkyl ester of (meth)acrylic acid, at least one C₁ to C₄ hydroxyalkyl ester of (meth)acrylic acid, and mixtures thereof. According to the present invention, the second monomer component b1) is selected from at least one monoethylenically unsaturated monomer containing at least one linear or branched C₁ to C₄ alkyl ester of (meth)acrylic acid, and mixtures thereof.

Suitable exemplary linear or branched C₁ to C₄ alkyl (meth)acrylate and hydroxyalkyl (meth)acrylate monomers set forth under the second monomer component b1) include methyl (meth)acrylate, ethyl (meth)acrylate, n-propyl (meth)acrylate, iso-propyl (meth)acrylate, n-butyl (meth)acrylate, isobutyl (meth)acrylate, t-butyl (meth)acrylate, hydroxyethyl (meth)acrylate, hydroxypropyl (meth)acrylate, hydroxybutyl (meth)acrylate (butane diol mono(meth)acrylate), and mixtures thereof.

The alkyl and hydroxyalkyl (meth)acrylate monomers set forth under the second monomer component b1) may be utilized in an amount ranging from 90% to 15% by weight, preferably from 80% to 25% by weight, more preferably from 65% to 35% by weight, based upon the total weight of the monomers in the polymerizable monomer composition.

The third monomer component c1) comprises a mixture of crosslinking monomers selected from at least two different polyunsaturated monomer types or classes. The first crosslinking monomer is selected from at least one polyfunctional acrylate having at least two polymerizable ethylenically unsaturated double bonds. The term "polyfunctional acrylate" refers to acrylate esters of organic polyols wherein the organic polyol is esterified by reacting it with (meth)acrylic acid. The polyfunctional acrylate can contain from 2 to 6 polymerizable ethylenically unsaturated double bonds. Suitable polyols for the esterification reaction can contain from 2 to 12 carbon atoms and have at least two hydroxyl groups. The polyol can be linear and branched or cyclic.

Exemplary polyols suitable for esterification may include, but are not limited to, alkylene glycols containing from 2 to 5 carbon atoms, polyalkylene glycol dimers and trimers, trimethylolethane and dimers thereof, trimethylolpropane and dimers thereof, triethylolpropane and dimers thereof, tetramethylolmethane (pentaerythritol), dipentaerythritol, and 1,4-cyclohexanediol.

Exemplary polyfunctional acrylates include, but are not limited to, ethylene glycol di(meth)acrylate, triethylene glycol di(meth)acrylate, 1,3-butylene glycol di(meth)acrylate, 1,4-butylene glycol di(meth)acrylate, 1,6-hexanediol di(meth)acrylate, neopentyl glycol di(meth)acrylate, 1,9-nonanediol di(meth)acrylate, trimethylolpropane tri(meth)acrylate, trimethylolethane tri(meth)acrylate, tetramethylolmethane tri(meth)acrylate, ditrimethylolpropane tetra(meth)acrylate, tetramethylolmethane tetra(meth)acrylate, pentaerythritol tetra(meth)acrylate; dipentaerythritol hexa(meth)acrylate, 1,4-cyclohexanediol dimethyacrylate, and mixtures thereof.

The second crosslinking monomer may be selected from at least one polyalkenyl polyether having at least two polymerizable ethylenically unsaturated double bonds. The term "polyalkenyl polyether" refers to alkenyl ethers of organic polyols wherein the organic polyol is etherified by reacting it with alkenyl halide, such as allyl chloride or allyl bromide. The polyalkenyl polyether (e.g., polyallyl polyether) may contain from 2 to 8 polymerizable ethylenically unsaturated double bonds. Suitable polyols for the etherification reaction may contain from 2 to 12 carbon atoms and have at least two hydroxyl groups. The polyol may be linear and branched or cyclic (e.g., monosaccharides and polysaccharides containing 1 to 4 saccharide units).

Exemplary polyols suitable for etherification may include, but are not limited to, glucose, galactose, fructose, sorbose, rhamnose, sucrose, arabinose, maltose, lactose, raffinose, pentaerythritol, dipentaerythritol, trimethylolethane and dimers thereof, trimethylolpropane and dimers thereof, and triethylolpropane and dimers thereof. Additional polyols suitable for the etherification are disclosed in U. S. Patent No. 2,798,053.

Exemplary polyalkenyl polyethers may include, but are not limited to, polyallyl ethers of sucrose having from 2 to 8 allyl groups per molecule, pentaerythritol diallyl ether, pentaerythritol triallyl ether, and pentaerythritol tetraallyl ether; trimethylolpropane diallyl ether, trimethylolpropane triallyl ether, and mixtures thereof.

The mixture of crosslinking monomers (first and second crosslinking monomer components) set forth under the third monomer component c1) may be present in an amount ranging from 0.01% to 5% by weight, preferably from 0.03% to 3% by weight, more preferably from 0.05% to 1 % by weight, based upon the total weight of the monomers in the polymerizable monomer composition.

The weight ratio of the first crosslinking monomer to the second crosslinking monomer in the polymerizable monomer mixture may range from 1:99 to 99:1, preferably from 1:9 to 9:1, more preferably from 2:8 to 8:2, more preferably from about 4:6 to about 6:4.

The optional fourth monomer component d1) may be a monoethylenically unsaturated monomer different from monomer components a1), b1), and c1).

Exemplary ethylenically unsaturated monomers of the optional fourth monomeric component d1) may include ethyl diglycol (meth)acrylate, 2-carboxyethyl (meth)acrylate, n-hexyl (meth)acrylate, 2-ethylhexyl (meth)acrylate, octyl (meth)acrylate, decyl (meth)acrylate, 6-hydroxyhexyl (meth)acrylate, 10-hydroxydecyl (meth)acrylate, styrene, α-methyl styrene, acrylonitrile, methacrylonitrile, acrylamide, methacrylamide, N,N'-dimethylaminoacrylamide, t-butylacrylamide, t-octylacrylamide, N-vinyl pyrrolidone, 2-acrylamido-2-methylpropane sulfonic acid, vinyl acetate, vinyl propionate, vinyl butanoate, vinyl valerate, vinyl hexanoate, vinyl octanoate, vinyl nonanoate, vinyl decanoate, vinyl neodecanoate, vinyl undecanoate, vinyl laurate, ACE(TM) and (M)ACE(TM) monomer available from Hexion Specialty Chemicals, Inc., Columbus, OH; and mixtures thereof. The foregoing monomers are commercially available and/or can be synthesized by procedures well known in the art, or as described herein.

As one of ordinary skill in the art will readily recognize the amounts of each of the first, second, third, and optional fourth monomer components (a1-d1) set forth herein will be selected from the disclosed ranges such that the sum of each of the monomer components in the polymerizable monomer compositions is equal to 100 wt.%.

Hence, the film-forming polymer of the adhesive composition may be a crosslinked acrylic copolymer which is obtained by polymerization from a monomer composition comprising - a first monomer component a1) being (meth)acrylic acid;
- a second monomer component b1) selected from at least one monoethylenically unsaturated monomer containing at least one linear or branched C₁ to C₄ alkyl ester of (meth)acrylic acid, and mixtures thereof;
- a third monomer component c1) comprising a mixture of crosslinking monomers selected from a first and second crosslinking monomer, wherein the first crosslinking monomer is selected from at least one polyfunctional acrylate having at least two polymerizable ethylenically unsaturated double bonds, and the second crosslinking monomer is selected from at least one polyalkenyl polyether having at least two polymerizable ethylenically unsaturated double bonds; and
- an optional fourth monomer component d1), which is a monoethylenically unsaturated monomer different from the first, second and third monomer components a1), b1), and c1).

An example of an acrylic copolymer for the adhesive composition may especially be the acrylic polymer known under the INCI name Polyacrylate-14 and sold under the trade name Fixate™ PLUS polymer, which is a slightly crosslinked acrylic copolymer comprising at least (meth)acrylic acid and C₁₋₄ alkyl(meth)acrylate, as the respective first and second monomer components a1) and b1).

The adhesive composition may comprise a total amount of acrylic copolymer ranging from 10% to 75%, preferably from 15% to 65%, more preferably from 20% to 60%, even more preferably from 25% to 50% by total weight of the adhesive composition.

The adhesive composition has a viscosity of from 0, 1 Pa.s to 75 Pa.s, preferably from 0,4 Pa.s to 3 Pa.s according to the Viscosity Test Method.

The adhesive composition may comprise a total amount of acrylic copolymer ranging from 10% to 75%, by total weight of the adhesive composition and the adhesive composition may have a viscosity of 0,1 Pa.s to 75 Pa.s according to the Viscosity Test Method.

The adhesive composition may preferably comprise a total amount of acrylic copolymer ranging from 20% to 60% by total weight of the adhesive composition, and the adhesive composition may have a viscosity of from 0,4 Pa.s to 3 Pa.s according to the Viscosity Test Method.

As described hereinafter, an Example 13 of the adhesive composition comprising Fixate™ PLUS polymer as the film-forming polymer has been prepared and used according to the method of the present invention.

### Cosmetically acceptable carrier

The adhesive composition comprises a cosmetically acceptable carrier. The cosmetically acceptable carrier of the adhesive composition may be an aqueous carrier. The adhesive composition may comprise water. Water can provide a hydrophilic phase, which the hydrophilic portions of any other ingredients comprised in the adhesive composition can interact with water. Water can also provide a fluid phase meaning that the adhesive composition can be in liquid form and therefore easily mixed with other fluid formulations such as the oxidizing formulation. The adhesive composition may comprise from 50% to 85% water, or from 65% to 75% of water by total weight of the composition.

The cosmetically acceptable carrier is any carrier suitable for formulating the film-forming polymer into an adhesive composition being suitable for application onto hair. The cosmetically acceptable carrier may be selected from either an aqueous medium or an aqueous-alcoholic medium. When the cosmetically acceptable carrier is an aqueous-alcoholic carrier, the cosmetically acceptable carrier may comprise water and an alcohol. An alcohol can advantageously influence the viscosity of a relatively wide spectrum of ingredients of the composition. The alcohol of the adhesive composition may be selected from the group consisting of ethanol, isopropanol, propanol, and mixtures thereof.

When the cosmetically acceptable carrier is an aqueous carrier, the aqueous carrier may consist essentially of water and may be substantially free of alcohol. The adhesive composition may comprise a safe and effective amount of a cosmetically acceptable carrier which is water. The adhesive composition may comprise from 0.1% to 99%, or from 1% to 98%, or from 10% to 97%, or from 30% to 95% of water by total weight of the composition.

When used daily, organic solvents in adhesive compositions may have a negative effect on humans and on the environment. They also have an unpleasant odor. Hence, the adhesive composition may be substantially free of organic solvents. Organic solvents may be any volatile alcohols, e.g. ethanol, isopropanol, propanol.

The adhesive composition may be substantially free of alcohol, such as volatile alcohols (e.g. ethanol, isopropanol, propanol). When the adhesive composition is substantially free of alcohol, the adhesive composition can advantageously have a reduced odour. Flammability issues can also be prevented.

The cosmetically acceptable carrier of the adhesive composition may be an oily compound. The oily compound may be selected from the group consisting of cyclic silicones and volatile hydrocarbons. Cyclic silicones can be available from Dow Corning. The cyclic silicone may have from at least 3 silicone atoms or from at least 5 silicone atoms but no more than 7 silicone atoms or no more than 6 silicone atoms. The cyclic silicone may conform to the formula: 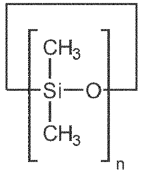 wherein n is from 3 or from 5 but no more than 7 or no more than 6. The cyclic silicone may have a kinematic viscosity of less than 10 cPs at 23°C. A Suitable cyclic silicone for use herein may include Cyclomethicone D5 (commercially available from G.E. Silicones). Alternatively, the adhesive composition may be silicone-free.

Volatile hydrocarbons e.g. Isopar can be obtained from ExxonMobil Petroleum and Chemical. The oily compound may be a mineral oil. Trade names for suitable mineral oils include Benol, Blandol, Hydrobrite, Kaydol (Sonneborn LLC Refined Products), Chevron Superla White Oil (Chevron Products Company), Drakeol, Parol (Calumet Penreco LLC), Peneteck (Calumet Penreco LLC), Marcol, and Primol 352 (ExxonMobil Petroleum and Chemical).

### Optional features for the adhesive composition

The adhesive composition may have a pH from 5 to 8, preferably from 6 to 7.5. The adhesive composition may be provided in a cosmetically acceptable carrier such as water, or an alcohol such as ethanol or a mixture of ethanol and water. It is intended that the adhesive composition may preferably have a neutral pH to avoid any hydrolysis of the film-forming polymer, or any precipitation.

The adhesive composition may be substantially free of surfactants, or may comprise a total amount of surfactants ranging from 1% to 10%, preferably from 1.5% to 5% by total weight of the adhesive composition. Having the adhesive composition free of surfactants can help to prevent the film-forming polymer to swell when it is in contact with the hair coloring composition.

### Optional ingredients for the adhesive composition

The adhesive composition may also contain one or more additives chosen from nonionic, anionic, cationic and amphoteric polymers, ceramides and pseudo ceramides, vitamins and provitamins including panthenol, water-soluble and liposoluble, silicone or non-silicone sunscreens, sequestering agents, plasticizers, solubilizers, acidifying agents, basifying agents, neutralizers, thickeners, antioxidants, hydroxy acids, penetrating agents, perfume and preservatives.

A person skilled in the art will take care to select the optional additives and the amount thereof such that they do not harm the properties of the adhesive composition according to the present invention.

These additives may be present in the adhesive composition according to the invention in an amount ranging from 0% to 20% by weight relative to the total weight of the composition.

### Application of the adhesive composition to a first plurality of hair fiber portions

The method comprises the step of applying the adhesive composition to a first plurality of hair fiber portions. The adhesive composition may be applied to dried or wet hair fibers.

The first plurality of hair fiber portions may be selected from the group consisting of a plurality of tips of the hair fibers, a plurality of roots of the hair fibers, a plurality of parts of the hair fibers across the length of the respective hair fibers, the whole length of one or more hair fibers, and combinations thereof.

For the step (c) of applying the adhesive composition to the first plurality of hair fiber portions, the adhesive composition of the invention may be employed with an application tool or an application form for a relatively easy and overall or localized application. Hence, the adhesive composition may be applied to the first plurality of hair fiber portions by an application tool selected from the group consisting of a relatively slim nozzle applicator, a sponge brush applicator, a spatula, a relatively fine paint-brush applicator, and combinations thereof.

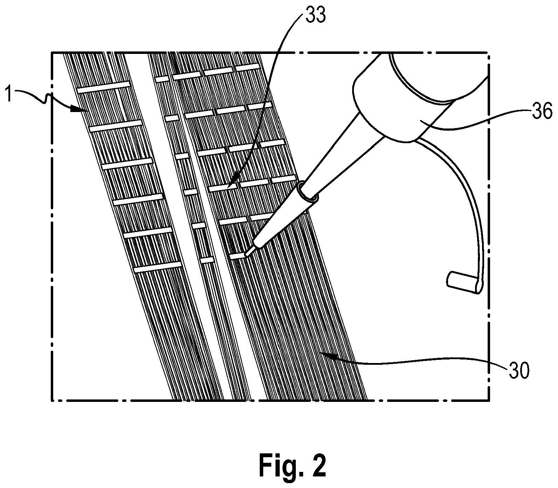

Fig. 2 is a schematic view of a first plurality of hair fiber portions on which an adhesive composition is applied by the means of an application tool comprising a relatively slim nozzle applicator. In Fig. 2, an adhesive composition is applied to a first plurality of hair fiber portions 30 to form a first pattern 33. The first pattern 33 may be in the form of regular stripes, for instance.

The relatively slim nozzle applicator may be a readily and precisely way to apply the adhesive composition to the first plurality of hair fiber portions. The relatively slim nozzle applicator tool allows the provision of filigree patterns as lines and dots.

The sponge brush applicator can be a tool to apply enough adhesive composition on the first plurality of hair fiber portions for providing a macro-sized pattern. Indeed, the sponge brush applicator can adhere to a sufficient amount of the adhesive composition, so that the application of the adhesive composition is relatively easy for relatively extensive and broad patterns.

The spatula may be another alternative to draw a plurality of fine lines as the first pattern. The most preferred application tool may be the relatively fine paint-brush applicator, all types of pattern of the adhesive composition may be thus achieved.

Without being limited, the first pattern may be a plurality of stripes, wavy lines, dots, and/or circles. The first pattern may comprise a plurality of elements having different geometries. One may position on the hair a tracing paper representing the contour of an image as the first pattern. Then, the adhesive composition may be applied using as the application form, a non-aerosol hair spray through the contours of the tracing image. Then, after application of the hair coloring composition and rinsing according to the method of the present invention, the hair fibers comprises the first pattern representing the image with the one or more initial color shades.

The adhesive composition may be applied to the first plurality of hair fiber portions for a time period ranging from 5 min to 30 min, preferably from 5 min to 15 min, more preferably from 10 min to 15 min. After applying the adhesive composition, the first plurality of hair fiber portions is dried.

The first plurality of hair fiber portions may be dried with a hairdryer for a time period ranging from 5 min to 30 min, preferably from 5 min to 15 min, more preferably from 10 min to 15 min. The period of time may vary according to the concentration of the film-forming polymer in the adhesive composition. The more the film-forming polymer is present in the adhesive composition, the less time it is needed to dry the first plurality of hair fiber portions.

The drying may be carried out by a hair dryer, or a blow dryer, or a hood device. Hair dryer or blow dryer distances between device and heads may be typically at a distance of 20 or 30 or 40 centimeters, at a temperature between 50°C and 100°C.

The adhesive composition may be applied according to known formulations such as a liquid aqueous solution, a gel, or a foam, or an aerosol; or a non-aerosol hair spray especially for macro-patterns.

When the adhesive composition is present in the form of a gel, at least one gel-forming substance is present in it in an amount of preferably from 0.05% to 10%, especially preferably from 0.1% to 2%, by weight of the adhesive composition. The viscosity of the gel may amount to, preferably, from 0,5 Pa.s to 50 Pa.s especially preferably from 1 Pa.s to 15 Pa.s at 25°C (measured with a rotary viscometer, RheoStress 100 of Haake at a temperature of 25°C and a shear rate of 0.5 to 1400 s⁻¹).

When the adhesive composition is in the form of a foam, at least one common foam-forming substance known for that purpose is included in it. The adhesive composition may be foamed with or without the aid of a propellant gases or chemical propellants and worked into the hair as a foam which loads the hair without being rinsed out. The adhesive composition may be then used with a device for foaming the adhesive composition. Those devices, which permit the foaming of a liquid with or without the use of a propellant, are suitable as foam-forming devices. Suitable mechanical foaming devices can be employed, for example a commercial foam pump or an aerosol foam head.

The aerosol hair spray may comprise a pressurized container, the adhesive composition which is contained in the pressurized container together with a propellant, and a spraying device connected with the pressurized container for dispensing the adhesive composition from the pressurized container.

When the adhesive composition is present in the form of an aerosol hair spray, it contains from 15% to 85% by weight, preferably from 25% to 75% by weight, of a propellant and is filled in a pressurized container. Lower alkanes, for example, such as n-butane, iso-butane and propane, or their mixtures, dimethyl ether, fluorinated hydrocarbons, such as F-152a (1,1-difluoroethane) or F-134 (tetrafluoroethane) and also propellants present in the form of compressed gases, are suitable as the propellant contained in the aerosol hair spray. Propane and butane may be especially preferred as the propellants. The solvent may be preferably purely alcoholic, but can also contain up to 10% by weight, preferably from 2% to 8%, by weight water.

When the adhesive composition is in the form of a non-aerosol hair spray, it is sprayed with the help of a suitable mechanically driven spraying device. The term "mechanical spraying devices" means those devices, which permit the spraying of a composition without the use of a propellant. These mechanical spraying devices include a spray pump or an elastic container provided with a spray valve. The adhesive composition is filled under pressure into the elastic container, which stretches so that the adhesive composition will be dispensed when the spray valve is opened so that the elastic container contracts.

### Provision of a hair coloring composition

The method comprises providing a hair coloring composition for forming a final color shade, which differ from the one or more initial color shades.

The hair coloring composition may comprise a dye composition and optionally a developer composition. The developer composition may comprise one or more oxidizing agents.

The dye composition may comprise one or more oxidative dye precursors comprising one or more couplers and one or more primary intermediates or the dye composition may consist essentially of one or more direct dyes.

### Oxidative dye precursors

The dye composition according to the present invention may comprise oxidative dye precursors, which are usually classified either as primary intermediates (also known as developers) or couplers (also known as secondary intermediates). Various couplers may be used with primary intermediates in order to obtain different shades. Oxidative dye precursors may be free bases or the cosmetically acceptable salts thereof.

Hence, the dye composition may comprise oxidative dyes precursors comprising one or more couplers (also known as secondary intermediate) and one or more primary intermediates (also known as developer).

The oxidative dye precursors suitable for use herein, in so far as they are bases, may be used as free bases or in the form of any cosmetically acceptable salts obtained with the corresponding organic or inorganic acids, such as hydrochloric, hydrobromic, citric, acetic, lactic, succinic, tartaric, or sulfuric acids, or, in so far as they have aromatic hydroxyl groups, in the form of any cosmetically acceptable salts obtained with the corresponding bases, such as alkali phenolates.

Oxidative dye precursors are known in the art, and include aromatic diamines, aminophenols, aromatic diols and their derivatives (a representative but not exhaustive list of oxidation dye precursors can be found in Sagarin, "Cosmetic Science and Technology, Interscience, Special Edn. Vol. 2 pages 308 to 310). Suitable oxidative dye precursors are also disclosed in the Canadian Patent Application No. CA2576189A1 - in particular, from Table 1 dye combinations No. 1 to 2394, which span pages 49 to 238, are incorporated herein by reference. It is to be understood that the one or more primary intermediates and the one or more couplers (collectively known as oxidative dye precursors) detailed below are only by way of example and are not intended to limit the compositions and other aspects herein described. The one or more primary intermediates and the one or more couplers may be used in the form of any cosmetically acceptable salts, for example sulfate salts.

Typically, the dye composition may comprise a total amount of oxidative dye precursors ranging up to 12%, from 0.001% to 12%, preferably from 0.1% to 10%, more preferably from 0.3% to 8%, even more preferably from 0.5% to 6%, by total weight of the composition.

The one or more primary intermediates may be selected from the group consisting of toluene-2,5-diamine, p-phenylenediamine, *N*-phenyl-p-phenylenediamine, *N,N*-bis(2-hydroxyethyl)-p-phenylenediamine, 2-hydroxyethyl-p-phenylenediamine, hydroxypropyl-bis-(N-hydroxyethyl-p-phenylenediamine), 2-methoxymethyl-p-phenylenediamine, 2-(1,2-dihydroxyethyl)-p-phenylenediamine, 2,2'-(2-(4-aminophenylamino)ethylazanediyl)diethanol, 2-(2,5-diamino-4-methoxyphenyl)propane-1,3-diol, 2-(7-amino-2H-benzo[b][1,4]oxazin-4(3H)-yl)ethanol, 2-chloro-p-phenylenediamine, p-aminophenol, p-(methylamino)phenol, 4-amino-m-cresol, 6-amino-m-cresol, 5-ethyl-o-aminophenol, 2-methoxy-p-phenylenediamine, 2,2'-methylenebis-4-aminophenol, 2,4,5,6-tetraminopyrimidine, 2,5,6-triamino-4-pyrimidinol, 1-hydroxyethyl-4,5-diaminopyrazole sulfate, 4,5-diamino-1-methylpyrazole, 4,5-diamino-1-ethylpyrazole, 4,5-diamino-1-isopropylpyrazole, 4,5-diamino-1-butylpyrazole, 4,5-diamino-1-pentylpyrazole, 4,5-diamino-1-benzylpyrazole, 2,3-diamino-6,7-dihydropyrazolo[1,2-a]pyrazol-1(5H)-one dimethosulfonate, 4,5-diamino-1-hexylpyrazole, 4,5-diamino-1-heptylpyrazole, methoxymethyl-1,4-diaminobenzene, N,N-bis(2-hydroxyethyl)-N-(4-aminophenyl)-1,2-diaminothane, 2-[(3-aminopyrazolo[1,5-a]pyridin-2-yl)oxy]ethanol hydrochloride, their salts thereof and mixtures thereof.

The one or more primary intermediate of the dye composition may be particularly 1,4-diamino-2-(methoxymethyl)-benzene. 1,4-diamino-2-(methoxymethyl)-benzene has the advantage of an improved sensitisation profile (i.e. reduced risks of scalp skin reaction).

The one or more primary intermediate may be 4,5-diamino-1-hexylpyrazole. 4,5-diamino-1-hexylpyrazole may be used as a sulfate salt.

The one or more primary intermediate may be selected from the group consisting of 4,5-diamino-1-butylpyrazole, 4,5-diamino-1-pentylpyrazole, 4,5-diamino-1-benzylpyrazole, 2,3-diamino-6,7-dihydropyrazolo[1,2-a]pyrazol-1(5H)-one dimethosulfonate, 4,5-diamino-1-hexylpyrazole, 4,5-diamino-1-heptylpyrazole, methoxymethyl-1,4-diaminobenzene, and mixtures thereof; and the cosmetically acceptable salts thereof such as chlorides, sulfates and hemi-sulfates in particular.

The one or more couplers may be a compound comprising one or more phenyl rings substituted with one or more hydroxyl groups.

The one or more couplers may be selected from the group consisting of resorcinol, 4-chlororesorcinol, 2-chlororesorcinol, 2-methylresorcinol, 4,6-dichlorobenzene-1,3-diol, 2,4-dimethylbenzene-1,3-diol, *m*-aminophenol, 4-amino-2-hydroxytoluene, 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, 6-hydroxybenzomorpholine, 2-amino-5-ethylphenol, 2-amino-5-phenylphenol, 2-amino-5-methylphenol, 2-amino-6-methylphenol, 2-amino-5-ethoxyphenol, 5-methyl-2-(methylamino)phenol, 2,4-diaminophenoxyethanol, 2-amino-4-hydroxyethylaminoanisole, 1,3-bis-(2,4-diaminophenoxy)-propane, 2,2'-(2-methyl-1,3-phenylene)bis(azanediyl)diethanol, benzene-1,3-diamine, 2,2'-(4,6-diamino-1,3-phenylene)bis(oxy)diethanol, 3-(pyrrolidin-1-yl)aniline, 1-(3-(dimethylamino)phenyl)urea, 1-(3-aminophenyl)urea, 1-naphthol, 2-methyl-1-naphthol, 1,5-naphthalenediol, 2,7-naphthalenediol or 1-acetoxy-2-methylnaphthalene, 4-chloro-2-methylnaphthalen-1-ol, 4-methoxy-2-methylnaphthalen-1-ol, 2,6-dihydroxy-3,4-dimethylpyridine, 2,6-dimethoxy-3,5-pyridinediamine, 3-amino-2-methylamino-6-methoxypyridine, 2-amino-3-hydroxypyridine, 2,6-diaminopyridine, pyridine-2,6-diol, 5,6-dihydroxyindole, 6-hydroxyindole, 5,6-dihydroxyindoline, 3-methyl-1-phenyl-1*H*-pyrazol-5(4*H*)-one, 1,2,4-trihydroxybenzene, 2-(benzo[d][1,3]dioxol-5-ylamino)ethanol (also known as hydroxyethyl-3,4-methylenedioxyaniline), and mixtures thereof.

The oxidative dye precursors may be particularly selected from the group consisting of 1-naphthol, 2,4-diaminophenoxyethanol, toluene-2,5-diamine sulfate, resorcinol, 4-amino-m-cresol, 2-amino-6-chloro-4-nitrophenol, 2-amino-4-hydroxyethylaminoanisole sulfate, hydroxyethyl-3,4-methylenedioxyaniline HCl, 1-hydroxyethyl 4,5-diamino pyrazole sulfate, 4-amino-2-hydroxytoluene, 2-methylresorcinol, m-aminophenol, 2-methyl-5-hydroxyethylaminophenol, and mixtures thereof.

The oxidative dye precursors may comprise preferably 5-amino-4-chloro-o-cresol and 1,4-diamino-2-(methoxymethyl)-benzene. The oxidative dye precursors may comprise more preferably 2,6-diaminopyridine and 1,4-diamino-2-(methoxymethyl)-benzene. The oxidative dye precursors may comprise even more preferably 2,6-dihydroxyethylaminotoluene and 2-methoxymethyl-1,4-diaminobenzene. The oxidative dye precursors may comprise even more preferably 2-methoxymethyl-1,4-diaminobenzene and *p*-phenylenediamine and/or toluene-2,5-diamine.

When the hair coloring composition of the invention is obtained by mixing a dye composition and a developer composition, the primary intermediates and couplers are usually incorporated into the dye composition.

### Direct dyes

The dye composition of the hair coloring composition may further comprise beyond one or more one or more oxidative dye precursors, one or more direct dyes, preferably one or more oxidatively stable direct dyes. Typically, the dye composition may comprise a total amount of direct dyes ranging from 0.001% to 4%, preferably from 0.005% to 3%, more preferably from 0.01% to 2%, by total weight of the composition.

Alternatively, the dye composition may preferably consist essentially of one or more direct dyes.

The presence of one or more direct dyes and the proportion thereof can help to provide or enhance coloring/dyeing, particularly with regard to the vibrancy of the color that is desired.

The dye composition may be substantially free of any direct dyes. Indeed, sometimes consumers prefer direct dye-free compositions.

The one or more direct dyes may be selected from the group consisting of nitro dyes to provide a blue color, nitro dyes to provide either a red color or a yellow color, quinone dyes, basic dyes, neutral azo dyes, acid dyes, and mixtures thereof. The one or more direct dyes may be a basic dye. The one or more direct dyes may be a neutral azo dye. The one or more direct dyes may be an acid dye.

The one or more direct dyes may be selected from the group consisting of Acid dyes such as Acid Yellow 1, Acid Orange 3, Acid Black 1, Acid Black 52, Acid Orange 7, Acid Red 33, Acid Yellow 23, Acid Blue 9, Acid Violet 43, HC Blue 16, Acid Blue 62, Acid Blue 25, Acid Red 4; Basic Dyes such as Basic Brown 17, Basic Red 118, Basic Orange 69, Basic Red 76, Basic Brown 16, Basic Yellow 57, Basic Violet 2, Basic Violet 14, Basic Blue 7, Basic Blue 26, Basic Red 2, Basic Blue 99, Basic Yellow 29, Basic Red 51, Basic Orange 31, Basic Yellow 87, Basic Blue 124, 4-(3-(4-amino-9,10-dioxo-9,10-dihydroanthracen-1-ylamino)propyl)-4-methylmorpholin-4-ium-methylsulfate, (*E*)-1-(2-(4-(4,5-dimethylthiazol-2-yl)diazenyl)phenyl)(ethyl)amino)ethyl)-3-methyl-1H-imidazol-3-ium chloride, (*E*)-4-(2-(4-(dimethylamino)phenyl)diazenyl)-1-methyl-1H-imidazol-3-ium-3-yl)butane-1-sulfonate, (*E*)-4-(4-(2-methyl-2-phenylhydrazono)methyl)pyridmium-1-yl)butane-1-sulfonate, N,N-dimethyl-3-(4-(methylamino)-9,10-dioxo-4a,9,9a,10-tetrahydroanthracen-1-ylamino)-N-propylpropan-1-aminium bromide; Disperse Dyes such as Disperse Red 17, Disperse Violet 1, Disperse Red 15, Disperse Black 9, Disperse Blue 3, Disperse Blue 23, Disperse Blue 377; Nitro Dyes such as 1-(2-(4-nitrophenylamino)ethyl)urea, 2-(4-methyl-2-nitrophenylamino)ethanol, 4-nitrobenzene-1,2-diamine, 2-nitrobenzene-1,4-diamine, Picramic acid, 2-hydroxyethyl Picramic acid, HC Red No. 13, 2,2'-(2-nitro-1,4-phenylene)bis(azanediyl)diethanol, HC Yellow No. 5, HC Red No. 7, HC Blue No.2, HC Yellow No. 4, HC Yellow No. 2, HC Orange No. 1, HC Red No. 1, 2-(4-amino-2-chloro-5-nitrophenylamino)ethanol, HC Red No. 3, 4-amino-3-nitrophenol, 4-(2-hydroxyethylamino)-3-nitrophenol, 2-amino-3-nitrophenol, 2-(3-(methylamino)-4-nitrophenoxy)ethanol, 3-(3-amino-4-nitrophenyl)propane-1,2-diol, HC Yellow No. 11, HC Violet No. 1, HC Orange No. 2, HC Orange No. 3, HC Yellow No. 9, HC Red No. 10, HC Red No. 11, 2-(2-hydroxyethylamino)-4,6-dinitrophenol, HC Blue No. 12, HC Yellow No. 6, HC Yellow No. 12, HC Blue No. 10, HC Yellow No. 7, HC Yellow No. 10, HC Blue No. 9, 2-chloro-6-(ethylamino)-4-nitrophenol, 6-nitropyridine-2,5-diamine, HC Violet No. 2, 2-amino-6-chloro-4-nitrophenol, 4-(3-hydroxypropylamino)-3-nitrophenol, HC Yellow No. 13, 6-nitro-1,2,3,4-tetrahydroquinoxaline, HC Red No. 14, HC Yellow No. 15, HC Yellow No. 14, N2-methyl-6-nitropyridine-2,5-diamine, N1-allyl-2-nitrobenzene-1,4-diamine, HC Red No. 8, HC Green No.1, HC Blue No. 14, HC Blue No. 16; Natural dyes such as Annato, Anthocyanin, Beetroot, Carotene, Capsanthin, Lycopene, Chlorophyll, Henna, Indigo, Cochineal; and mixtures thereof.

When the hair coloring composition is obtained by mixing a dye composition and a developer composition, the direct dyes are usually incorporated into the dye composition.

### Oxidizing agent

The developer composition according to the present invention may comprise one or more oxidizing agent(s). The oxidizing agent(s) may preferably be selected from the group consisting of hypochlorous acid, peracetic acid, persulfate, preferably sodium persulfate, ammonium persulfate, chlorine dioxide, perboric acid, their salts thereof, ozone, hydrogen peroxide and mixtures thereof. The oxidizing agent(s) may more preferably be selected from the group consisting of hypochlorous acid, their salts thereof and mixtures thereof. The oxidizing agent(s) may even more preferably be selected from the group consisting of sodium hypochlorite, calcium hypochlorite, potassium hypochlorite and mixtures thereof.

The developer composition of the present invention may comprise one or more oxidizing agents and/or one or more sources of an oxidizing agent. The one or more oxidizing agents may be provided in aqueous solution or as a powder which is dissolved prior to use. Preferred oxidizing agents are water-soluble peroxygen oxidizing agents. Suitable water-soluble oxidizing agents include, but are not limited to: inorganic peroxygen materials capable of yielding hydrogen peroxide in an aqueous solution.

The one or more oxidizing agents are valuable for the initial solubilisation and decolorisation of the melanin (bleaching) and accelerate the oxidation of the oxidative dye precursors (oxidative dyeing) in the hair shaft.

The developer composition may comprise a total amount of oxidizing agents ranging from 0.1% to 15%, alternatively from 0.2 % to 15%, alternatively from 0.3 % to 15%, alternatively ranging from 0.1% to 12%, alternatively from 0.2 % to 12%, alternatively from 0.3 % to 12%, alternatively from 0.1% to 7%, alternatively from 0.2% to 7%, alternatively from 0.3% to 7%, alternatively from 1% to 7%, alternatively from 0.1% to 5%, alternatively from 0.2% to 5%, alternatively from 0.3% to 5%, alternatively from 0.5% to 5%, alternatively from 1% to 5%, alternatively from 2% to 5%, by total weight of the composition.

Alternatively, the developer composition may comprise a total amount of oxidizing agents of less than 3%, alternatively less than 2%, alternatively less than 1%, alternatively less than 0.5%, alternatively less than 0.3% alternatively less than 0.1% by total weight of the composition. The lower limit for the oxidizing agents may be at least 0.01% by total weight of the composition.

The dye composition may also be substantially free of oxidizing agents, i.e. having oxidizing agents less than 0.1%, and more particularly less than 0.01% by total weight of the composition.

Suitable water-soluble peroxygen oxidizing agents include, but are not limited to: hydrogen peroxide; inorganic alkali metal peroxides (such as sodium periodate and sodium peroxide); organic peroxides (such as urea peroxide and melamine peroxide); inorganic perhydrate salt bleaching compounds (such as the alkali metal salts of perborates, percarbonates, perphosphates, persilicates, persulphates and the like); and mixtures thereof. Inorganic perhydrate salts may be incorporated for example as monohydrates, tetrahydrates. Alkyl/aryl peroxides and/or peroxidases may also be used. Mixtures of two or more such oxidizing agents can be used if desired.

The developer composition may comprise a water-soluble oxidizing agent selected from the group consisting of peroxides, percarbonates (which may be used to provide a source of both oxidizing agent and carbonate ions and or ammonium ions), persulphates, and mixtures thereof. The particularly preferred oxidizing agent is hydrogen peroxide.

When the hair coloring composition of the present invention is obtained by mixing a developer composition and a dye composition prior to use, the one or more oxidizing agents may be present in the developer composition. The developer composition may be based on any desired formulation chassis, including any commercial product, for example an oil-in-water emulsion. Typical developer compositions comprise 6% or 9% of the H₂O₂ relative to the total weight of the developer composition. A preferred example of a developer composition with respectively 6% and 9% H₂O₂, comprises as INCI ingredients: Water, H₂O₂, Cetearyl Alcohol, Ceteareth-25, Salicylic Acid, Phosphoric Acid, Disodium Phosphate, Etidronic Acid. Another preferred example a developer composition comprises as INCI ingredients: Water, H₂O₂, cetearyl alcohol, lanolin alcohol, sodium lauryl sulfate, parfum, salicylic acid, phosphoric acid, disodium phosphate, linalool, hexyl cinnamal, etidronic acid, tocopherol. Another preferred example a developer composition comprises as INCI ingredients: Water, H₂O₂, cetearyl alchohol, lanolin alcohol, sodium lauryl sulfate, parfum, salicylic acid, phosphoric acid, disodium phosphate, linalool, hexyl cinnamal, etidronic acid, tocopherol.

### Optional ingredients for the hair coloring composition

The hair coloring composition according to the present invention may comprise, in addition to the ingredients indicated above, further ingredients in order to further enhance the properties of the hair coloring composition, as long as these are not excluded by the claims.

Suitable further optional ingredients include, in a cosmetically acceptable carrier, but not limited to: solvents; alkalizing agents; pigments, colored material, chelants; radical scavengers; pH modifiers and buffering agents; thickeners and/or rheology modifiers; carbonate ion sources; peroxymonocarbonate ion sources; surfactants; fragrances; enzymes; dispersing agents; peroxide stabilizing agents; antioxidants; natural ingredients (such as proteins, protein compounds, and plant extracts); conditioning agents (such as silicones and cationic polymers); ceramides; preserving agents; opacifiers and pearling agents (such as titanium dioxide and mica); and mixtures thereof. The further optional ingredients may be present in the hair coloring composition in an amount ranging from 0% to 20% by weight, relative to the total weight of the hair coloring composition.

Suitable further ingredients referred to above, but not specifically described below, are listed in the International Cosmetics Ingredient Dictionary and Handbook, (8th ed.; The Cosmetics, Toiletry, and Fragrance Association). Particularly, vol. 2, sections 3 (Chemical Classes) and 4 (Functions), which are useful in identifying specific adjuvants to achieve a particular purpose or multipurpose. A few of these ingredients are discussed hereinbelow, whose disclosure is of course non-exhaustive.

### Pigment

The hair coloring composition, preferably the dye composition may comprise one or more pigments. The one or more pigments of the hair coloring composition, preferably the dye composition may be a colored pigment which imparts color effects to the composition or to the hair.

Alternatively, the one or more pigments of the hair coloring composition, preferably the dye composition may be a lustre effect pigment which imparts desirable and aesthetically pleasing lustre effects to the hair coloring composition or to the keratin fibers of the hair. The color or lustre effects on the keratin fibers of the hair may be preferably temporary. Indeed, the color or lustre effects on the keratin fibers of the hair last until the next hair wash and can be removed again by washing the hair with customary shampoos.

The hair coloring composition, preferably the dye composition may be substantially free of pigment. Indeed, having a composition substantially free of pigment can help to prevent the formation of residues, precipitation and/or rough hair feel.

The hair coloring composition, preferably the dye composition may comprise one or more pigments having a D₅₀ particle diameter of from 5 µm to 60 µm measured according to the following test method. Particle diameter is represented by D₅₀, which is the median diameter by volume. D₅₀ is measured with a Malvern Mastersizer 2000, which is a laser diffraction particle sizer and it is measured according to ISO 13320:2009(en) with Hydro 2000G or Hydro 2000S, wherein the dispersant is water or ethanol. Detection range is from 0.02 µm to 2000 µm. D₅₀ is expressed as x₅₀ in ISO 13320:2009(en). Laser diffraction measures particle size distributions by measuring an angular variation in intensity of light scattered as a laser beam passes through a dispersed particulate sample analyser and the particle size is reported as a volume equivalent sphere diameter. A discussion of calculating D₅₀ is provided in Barber et al, Pharmaceutical Development and Technology, 3(2), 153-161 (1998), which is incorporated herein by reference.

The hair coloring composition, preferably the dye composition may comprise a pigment having a D₅₀ particle diameter from 10 µm to 40 µm. The one or more pigments of the hair coloring composition may be present in the composition in an undissolved form. The hair coloring composition, preferably the dye composition may comprise from 0.01% to 25%, or from 0.1% to 20%, or from 1% to 15%, or from 4% to 10% of the one or more pigments by total weight of the composition.

The one or more pigments of the hair coloring composition, preferably the dye composition may be a colorant which is virtually insoluble in the composition, and may be inorganic or organic. Inorganic-organic mixed pigments may be also possible. The hair coloring composition, preferably the dye composition may comprise an inorganic pigment. The advantage of an inorganic pigment is its excellent resistance to light, weather and temperature. The inorganic pigment of the hair coloring composition may be of natural origin, and may be, for example, derived from a material selected from the group consisting of chalk, ochre, umber, green earth, burnt sienna, and graphite.

The one or more pigments of the hair coloring composition, preferably the dye composition may be a white pigment, such as, for example, titanium dioxide or zinc oxide. Alternatively, the one or more pigments of the hair coloring composition, preferably the dye composition may be a black pigment, such as, for example, iron oxide black. Alternatively, the one or more pigments of the composition may be a colored pigment, such as, for example, ultra-marine or iron oxide red, or a lustre pigment, or a metal effect pigment, or a pearlescent pigment, and/or a fluorescent or phosphorescent pigment.

The one or more pigments of the hair coloring composition, preferably the dye composition may be colored or a non-white pigment. The one or more pigments of the hair coloring composition, preferably the dye composition may be selected from the group consisting of metal oxides, hydroxides and oxide hydrates, mixed phase pigments, sulfur-containing silicates, metal sulfides, complex metal cyanides, metal sulfates, chromates and molybdates, the metals themselves (bronze pigments), and combinations thereof. The one or more pigments of the hair coloring composition, preferably the dye composition may be selected from the group consisting of titanium dioxide (CI 77891), black iron oxide (CI 77499), yellow iron oxide (CI 77492), red and brown iron oxide (CI 77491), manganese violet (CI 77742), ultramarine (sodium aluminium sulfosilicates, CI 77007, Pigment Blue 29), chromium oxide hydrate (CI 77289), Prussian blue (ferric ferrocyanide, CI 77510), carmine (cochineal), and combinations thereof.

The one or more pigments of the hair coloring composition, preferably the dye composition may be a pearlescent and colored pigment based on mica which is coated with a metal oxide or a metal oxychloride, such as titanium dioxide or bismuth oxychloride, and optionally further color-imparting substances, such as iron oxides, Prussian blue, ultramarine, and carmine. The color exhibited by the pigment may be adjusted by varying the layer thickness. Such pigments are sold, for example, under the trade names Rona®, Colorona®, Dichrona®, RonaFlair®, Ronastar®, Xirona® and Timiron® all of which are available from Merck, Darmstadt, Germany. For example, Xirona® is a brand for color travel pigments that display color shifting effects depending on the viewing angle and are based on either natural mica, silica or calcium aluminium borosilicate flakes, coated with varying layers of titanium dioxide.

Pigments from the line KTZ® from Kobo Products, Inc., 3474 So. Clinton Ave., So. Plainfield, USA, may be also useful herein, in particular the Surface Treated KTZ® Pearlescent Pigments from Kobo. Particularly useful are KTZ® FINE WHITE (mica and TiO₂) having a D₅₀ particle diameter from 5 µm to 25 µm and also KTZ® CELESTIAL LUSTER (mica and TiO₂, from 10 µm to 60 µm) as well as KTZ® CLASSIC WHITE (mica and TiO₂, from 10 µm to 60 µm). Another useful pigment may be SynCrystal Sapphire from Eckart Effect Pigments, which is a blue powder comprising platelets of synthetic fluorphlogopite coated with titanium dioxide, ferric ferrocyanide and small amounts of tin oxide. Another useful pigment may also be SYNCRYSTAL Almond also from Eckart, which is a beige powder with a copper reflection color and is composed of platelets of synthetic fluorphlogopite and coated with titanium dioxide and iron oxides. Another useful pigment may be Duocrome® RV 524C from BASF, which provides a two color look via a lustrous red powder with a violet reflection powder due to its composition of mica, titanium dioxide and carmine.

The one or more pigments of the hair coloring composition, preferably the dye composition may be an organic pigment. The organic pigment of the hair coloring composition may be selected from the group consisting of natural pigments sepia, gamboge, bone charcoal, Cassel brown, indigo, chlorophyll and other plant pigments.

The one or more pigments of the hair coloring composition, preferably the dye composition may be a synthetic organic pigment. The synthetic organic pigment of the hair coloring composition may be selected from the group consisting of azo pigments, anthraquinoids, indigoids, dioxazine, quinacridone, phthalocyanine, isoindolinone, perylene and perinone, metal complex, alkali blue, diketopyrrolopyrrole pigments, and combinations thereof.

The one or more pigments of the hair coloring composition, preferably the dye composition may be selected from the group consisting of iron oxide, titanium dioxide, mica, borosilicate, and combinations thereof. The pigment of the hair coloring composition may comprise an iron oxide (Fe₂O₃) pigment. The one or more pigments of the hair coloring composition may comprise a combination of mica and titanium dioxide.

### Colored material

The hair coloring composition, preferably the dye composition may comprise one or more colored materials. The one or more colored materials of the hair coloring composition, preferably the dye composition may be particulate in form. The one or more colored materials of the hair coloring composition, preferably the dye composition may be selected from the group consisting of colored fibers, colored beads, colored particles such as nano-particles, colored polymers comprising covalently attached dyes, liquid crystals, particles having diffraction properties, UV absorber and photoprotective substances, pressure- or light-sensitive pigments, and combinations thereof.

The hair coloring composition, preferably the dye composition may be substantially free of colored material. Indeed, having a hair coloring composition substantially free of colored material can help to prevent the formation residues and precipitation.

The one or more colored materials of the hair coloring composition, preferably the dye composition may be capable of changing color via a mechanism selected from the group consisting of thermochromism, photochromism, hydrochromism, magnetochromism, electrochromism, piezochromism, chemichromism, mechano-optics. Suitable colored material of the hair coloring composition may include 3D Magnetic Pigments, Glow Dust, Fluorescent Pigments, Thermo Dust, Chameleon Pigments and other color changing materials from Solar Color Dust (http://solarcolordust.com/).

The hair coloring composition, preferably the dye composition may comprise one or more photoprotective substances. The hair coloring composition, preferably the dye composition may comprise from 0.01% to 10%, or from 0.1% to 5%, or from 0.2% to 2% of the one or more photoprotective substances by total weight of the composition. Useful photoprotective substances of the composition are specified in European Patent Application 1084696A1 from §0036 to §0053, which is incorporated herein by reference. The one or more photoprotective substances of the composition may be selected from the group consisting of 2-ethylhexyl 4-methoxycinnamate, methyl methoxycinnamate, 2-hydroxy-4-methoxybenzophenone-5-sulfonic acid, polyethoxylated p-aminobenzoates, di-butyl-hydroxytoluene (BHT), and mixtures thereof.

The hair coloring composition, preferably the dye composition may comprise from 0.01% to 10%, or from 0.05% to 5% of one or more particulate substances by total weight of the composition. The one or more particulate substances of the hair coloring composition may be a substance which is solid at room temperature (23°C) and in the form of a particle. The one or more particulate substances of the hair coloring composition, preferably the dye composition may be selected from the group consisting of silica, silicates, aluminates, clay earths, mica, and insoluble salts. The one or more particulate substances of the hair coloring composition, preferably the dye composition may be selected from the group consisting of insoluble inorganic metal salts, metal oxides, minerals and insoluble polymer particles. The one or more particulate substances of the hair coloring composition, preferably the dye composition may be titanium dioxide.

The one or more particulate substances of the hair coloring composition, preferably the dye composition may be present in the hair coloring composition, preferably the dye composition in an undissolved, or a stably dispersed form, and, following application to the hair and evaporation of the solvent, can deposit on the hair in a solid form.

The one or more particulate substances of the hair coloring composition, preferably the dye composition may be selected from the group consisting of silica (silica gel, silicon dioxide) and metal salts, in particular inorganic metal salts. The particulate substance of the hair coloring composition may be silica. The one or more particulate substances of the hair coloring composition, preferably the dye composition may be selected from the group consisting of metal salts such as alkali metal or alkaline earth metal halides, e.g. sodium chloride or potassium chloride; alkali metal or alkaline earth metal sulfates, such as sodium sulfate or magnesium sulfate.

### Application of the hair coloring composition to a second plurality of hair fiber portions

The method of the present invention comprises the step of applying the hair coloring composition to a second plurality of hair fiber portions including the first plurality of hair fiber portions comprising the first pattern, preferably to all the hair fibers.

The coloring step is related to color the hair fibers which are not protected by the first pattern comprising the adhesive composition. The second plurality of hair fiber portions may comprise from a portion of the hair including the first pattern to all the hair fibers of the hair. The method allows obtaining accurate patterns, and also while leaving some portions of the hair free of any treatments according to the method of the present invention.

Finally, the method comprises the step of rinsing the adhesive composition and the hair coloring composition out of the hair fibers, and drying the hair fibers, to form a second pattern. The second pattern comprises the second plurality of hair fiber portions without the first pattern, such that the hair fibers comprises the first pattern which has the one or more initial color shades and the second pattern which has the final color shade.

The hair coloring composition may be applied to the second plurality of hair fibers from 5 min to 40 min, preferably from 10 min to 30 min, more preferably from 15 min to 20 min. In other words, the second plurality of hair fiber portions may be left to stand with the hair coloring composition applied on the second plurality of hair fiber portions from 5 min to 40 min, preferably from 10 min to 30 min, more preferably from 15 min to 20 min. When applying the hair coloring composition for a relatively short time from 5 to 10 min, a glossing final shade is desired for the second pattern. When applying the hair coloring composition for a time from 30 to 40 min, a full coloration is desired for the second pattern.

Up to 40 minutes, the film-forming polymer coated on the first plurality of hair fibers may start to swell, however without no or at least enough dissolution of the film-forming polymer into the hair coloring composition such that the first plurality of hair fiber portions remain uncolored by the hair coloring composition. Hence, after the adhesive composition is applied to the first plurality of hair fiber portions, the first plurality of hair fiber portions is dried in order to prevent any swelling of the film-forming polymer in the next step of applying the hair coloring composition to the second plurality of hair fiber portions. As a precautious measure, the hair fibers may preferably be left to stand, i.e. may not be touched, shaken or moved, after having applied the hair coloring composition to the second plurality of hair fiber portions.

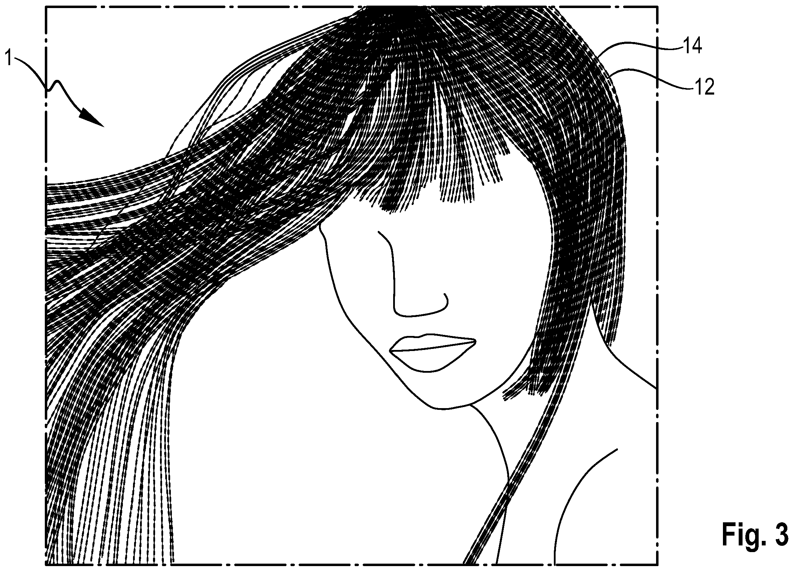

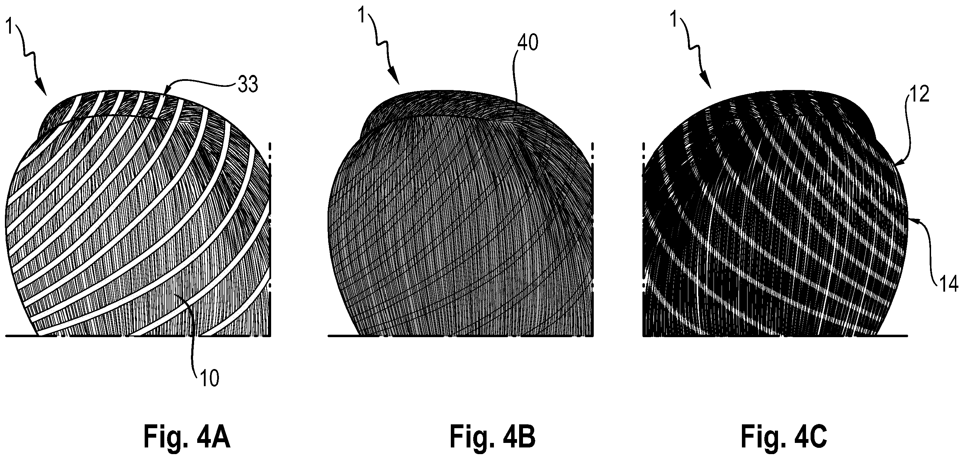

Fig. 3 is another schematic side view, showing the hair fibers 1 comprising a first pattern 12 which has the initial color shade and a second pattern 14 which has a final color shade. In order to obtain such result, the overall method can be illustrated by the following Figs. 4A-4C.

Fig. 4A illustrates a schematic view of the hair fibers 1 after application of an adhesive composition to the first plurality of hair fiber portions to form a first pattern 33. The first pattern 33 is a plurality of circular stripes around the head of a mannequin.

Fig. 4B illustrates a schematic view of the hair fibers 1 of Fig. 4A after application of a hair coloring composition 40 to all the hair fibers. After rinsing the adhesive composition and the hair coloring composition out of the hair fibers of Fig. 4B, and drying the hair fibers, Fig. 4C illustrates a schematic final view of the hair fibers 1 as Fig. 3 with the formation of a second pattern 14, wherein the second pattern 14 comprises all the hair fibers without the first pattern 12, such that the hair fibers comprises the first pattern 12 which has the initial color shade and the second pattern 14 which has the final color shade.

The Examples hereinafter have shown that when the adhesive composition comprised as the film-forming polymer, an acrylic copolymer as Fixate™ Plus Polymer, or an aryl-sulfonated polyester as Eastman AQ™ 38S Polymer or Eastman AQ™ 2350 Polymer, the adhesive composition is able to cover and temporary protect the first plurality of hair fiber portions from the hair color composition subsequently applied to all the hair fibers, in comparison to the film-forming polymer Gaffix™ VC-713.

When the method of the present invention is used as described herein, relatively regular repetitive color patterns with relatively strong contours that blend together when hair moves have been successfully obtained. The method herein makes it possible to deliver a first and a second patterns with sharp well defined areas. These sharp well defined areas may be geometrical patterns, especially striped patterns like it can be seen in feather, fur or fish.

### Bleaching step

The method of the present invention may further comprise a step of bleaching the hair between steps (d) and (e), wherein the step of bleaching is achieved by using one or more bleaching oxidizing agents selected from the group consisting of hydrogen peroxide, persulfate, perboric acid, peracetic acid, their salts thereof, and mixtures thereof.

Hence, the film-forming polymer of the adhesive composition can help to block the bleaching oxidizing agents to bleach the first plurality of hair fiber portions.

### KIT

A second aspect of the present invention is a kit for coloring hair fibers into a first and a second patterns, preferably human hair fibers, comprising
(a) an adhesive composition,
   wherein the adhesive composition comprises, in a cosmetically acceptable carrier, a film-forming polymer in a total amount of film-forming polymer ranging from 10% to 75%, preferably from 15% to 70%, more preferably from 20% to 60%, even more preferably from 25% to 50% by total weight of the adhesive composition;
      wherein the film-forming polymer is water-soluble or water-dispersible at 25°C;
   wherein the film-forming polymer has a glass transition temperature from -20°C to 100°C, preferably from -20°C to 70°C, more preferably from 0°C to 65°C;
   wherein the adhesive composition has a viscosity of from 100 centipoises to 75 000 centipoises, preferably from 400 centipoises to 3 000 centipoises according to the Viscosity Test Method; and
   wherein the film-forming polymer is as defined in the appended claims;
(b) a dye composition, preferably comprising one or more oxidative dye precursors or consisting essentially of one or more direct dyes;
(c) optionally, a developer composition comprising one or more oxidizing agents;
   wherein the adhesive composition, the dye composition (b) and optionally the developer composition (c) are separately packaged in different containers or packaged in a same container in different compartments; and
(d) an instructional material for preparation, for use, or both preparation and use, of the adhesive composition, the dye composition and optionally the developer composition.

The instructional material can contain instructions how to use the adhesive composition to protect the first plurality of hair fiber portions according the method of the present invention. Also, the instructional material can contain further instructions how to prepare the hair coloring composition, and how to use the different compositions of the kit. The instructional material can also contain some explanations how to draw the first and the second patterns, with illustrations about the first and the second patterns.

The kit may further comprise a mixing receptacle and/or a mixing means. The mixing receptacle of the kit may be a bowl. The mixing means of the kit may be a spatula.

The dye composition and the developer composition may be packaged in separate sealed containers. The dye composition may be packaged in a flexible tube packaging composed of metal, plastics or a combination thereof. The developer composition may be packaged in a squeezable container. The squeezable container may have at least 50% headspace. The squeezable container may have a headspace being at least the volume of the hair coloring composition (a). The developer composition may be packaged in a plastic container according to claim 1 of European Patent Application EP 2 801 281 A1, wherein the plastic container has two symmetrical collapsible side panels and a non-collapsible squeezable back panel; wherein the ratio of the average thicknesses between front and/or back panels and the side panels is at least 2:1 (See EP 2 801 281 A1 from paragraphs [0025] to [0044] as well as the Figures). The plastic container has the advantage that it is resistant to random, uncontrolled deformation under a substantial pressure differential between the environment and inside the container, yet having an affordable cost of manufacture and/or being appealing to the consumer.

The adhesive composition in the kit may be supplied as a single-use disposable dose. The single-use disposable dose can help to avoid using a container containing the adhesive composition which might dry over the time.

### EXAMPLES

The following examples are non-limiting examples of the present invention. The examples are given solely for the purpose of illustration, and are not to be construed as limitations of the present invention since many variations thereof are possible without departing from the spirit and scope of the invention, which would be recognized by one of ordinary skill in the art. All concentrations are listed as weight percent (% wt.), unless otherwise specified. "QSP" means sufficient quantity for 100% or for 100 g.

### Adhesive compositions Comparative Example C1, and Examples I1, I2 and I3

The following adhesive compositions have been prepared.

| **Ingredients** | **Comp. Ex. C1** (% wt.) | **Ex. I1** (% wt.) | **Ex. I2** (% wt.) | **Ex. I3** (% wt.) |
|---|---|---|---|---|
| Film-forming polymer | Gaffix™ VC-713 | Eastman AQ™ 38S Polymer | Eastman AQ™ 2350 Polymer | Fixate™ Plus Polymer |
| | 15 | 15 | 15 | 20 |
| Aminomethyl propanol | 0 | 0 | 0 | 1.3 |
| Methylparaben | 0.3 | 0.3 | 0.3 | 0.3 |
| Propylparaben | 0.15 | 0.15 | 0.15 | 0.3 |
| Phenoxyethano l | 0.5 | 0.5 | 0.5 | 0.5 |
| PEG-40 hydrogenated Castor Oil | 1.0 | 1.0 | 1.0 | 1.0 |
| Water purified | QSP | QSP | QSP | QSP |

| | | | | |
|---|---|---|---|---|
| Gaffix™ VC-713 polymer is a terpolymer of vinyl caprolactam/vinylpyrrolidone/dimethylaminoethyl methacrylate, which is supplied by Ashland; Eastman AQ™ 38S Polymer is supplied by Eastman Chemical Company, and is a sulfopolyester, namely Polyester-5 (INCI name); Eastman AQ™ 2350 Polymer is supplied by Eastman Chemical Company, and is another sulfopolyester, namely Polyester-5 (INCI name); Fixate™ Plus Polymer is supplied by Lubrizol, and is a slightly cross-linked acrylate copolymer of (C₁-C₄ alkyl)(meth-)acrylate, (meth)acrylic acid, namely Polyacrylate-14 (INCI name). | | | | |

### Material needed

**Hair strands:** Art 811300-9, Color 9/0, Mixture 811
Virgin hair strands having a width of 1.4 cm and a length of 13 cm.
Available from *Kerling International Haarfabric GmbH, Backnang, Germany*
Mass: 1.15 g ± 0.10 g

The following examples according to the present invention have been prepared:

### Comparative Example 1

0.30 g of the adhesive composition Comparative Example C1 was applied to a first plurality of hair fiber portions of a 1.15 g of the above-identified blond (9/0) hair strand by using as an application tool to form a first pattern. The application tool was a bottle containing the adhesive composition and fitted with a relatively slim nozzle (see for instance Fig. 2). The first pattern was a plurality of regular and repetitive circular stripes. The hair strand was dried with a hair dryer for 20 min.

A hair coloring composition has been prepared by mixing a dye composition consisting of 10 g of Wella™ Koleston Perfect 66/46 (red color) and a developer composition consisting of 10 g of Wella™ Welloxon Perfect Developer Cream, 6% wt. hydrogen peroxide in 10 g of water.

The resulting hair coloring composition was applied to all the hair fibers of the dried hair strands. The hair strand was left to stand for 30 minutes at 25°C. After 30 minutes, the hair strand was rinsed for 2 min under tap water (6 L/min, 35°C) and then blow-dried.

After the adhesive composition and the hair coloring composition are rinsed off the hair for 2 minutes, the hair is then shampooed with Wella™ Professionals Brillance, Fine/Normal shampoo commercially available in Germany in April 2014 and conditioned with Wella™ Professionals Brillance, Fine/Normal conditioner commercially available in Germany in April 2014 for 1 minute and then blow-dried.

It has been obtained a hair strand having a first and a second patterns. The hair fibers comprised the first pattern (where the adhesive composition has been applied) which had not the initial blond (9/0) shade, but a slightly red shade; and the second pattern comprised all the hair fibers without the first pattern. The second pattern had the red color shade (66/46).

Hence, when the adhesive composition comprised as the film-forming polymer Gaffix™ VC-713, the adhesive composition was not able to temporary protect the first plurality of hair fiber portions from the hair color composition subsequently applied to all the hair fibers.

### Example 1

0.30 g of the adhesive composition Example I1 was applied to a first plurality of hair fiber portions of a 1.15 g of the above-identified blond (9/0) hair strand by using as an application tool to form a first pattern. The application tool was a bottle containing the adhesive composition and fitted with a relatively slim nozzle (see for instance Fig. 2). The first pattern was a plurality of regular and repetitive circular stripes. The hair strand was dried with a hair dryer for 20 min.

A hair coloring composition has been prepared by mixing a dye composition consisting of 10 g of Wella™ Koleston Perfect 66/46 (red color) and a developer composition consisting of 10 g of Wella™ Welloxon Perfect Developer Cream, 6% wt. hydrogen peroxide in 10 g of water.

The resulting hair coloring composition was applied to all the hair fibers of the dried hair strands. The hair strand was left to stand for 30 minutes at 25°C. After 30 minutes, the hair strand was rinsed for 2 min under tap water (6 L/min, 35°C) and then blow-dried.

After the adhesive composition and the hair coloring composition are rinsed off the hair for 2 minutes, the hair is then shampooed with Wella™ Professionals Brillance, Fine/Normal shampoo commercially available in Germany in April 2014 and conditioned with Wella™ Professionals Brillance, Fine/Normal conditioner commercially available in Germany in April 2014 for 1 minute and then blow-dried.

It has been obtained a hair strand having a first and a second patterns. The hair fibers comprised the first pattern (where the adhesive composition has been applied) which had the initial blond (9/0) shade; and the second pattern comprised all the hair fibers without the first pattern. The second pattern had the red color shade (66/46).

Hence, when the adhesive composition comprised as the film-forming polymer Eastman AQ™ 38S Polymer, the adhesive composition is able to cover and temporary protect the first plurality of hair fiber portions from the hair color composition subsequently applied to all the hair fibers.

### Example 2

0.30 g of the adhesive composition Example 12 was applied to a first plurality of hair fiber portions of a 1.15 g of the above-identified blond (9/0) hair strand by using as an application tool to form a first pattern. The application tool was a bottle containing the adhesive composition and fitted with a relatively slim nozzle (see for instance Fig. 2). The first pattern was a plurality of regular and repetitive circular stripes. The hair strand was dried with a hair dryer for 20 min.

A hair coloring composition has been prepared by mixing a dye composition consisting of 10 g of Wella™ Koleston Perfect 66/46 (red color) and a developer composition consisting of 10 g of Wella™ Welloxon Perfect Developer Cream, 6% wt. hydrogen peroxide in 10 g of water.

The resulting hair coloring composition was applied to all the hair fibers of the dried hair strands. The hair strand was left to stand for 30 minutes at 25°C. After 30 minutes, the hair strand was rinsed for 2 min under tap water (6 L/min, 35°C) and then blow-dried.

After the adhesive composition and the hair coloring composition are rinsed off the hair for 2 minutes, the hair is then shampooed with Wella™ Professionals Brillance, Fine/Normal shampoo commercially available in Germany in April 2014 and conditioned with Wella™ Professionals Brillance, Fine/Normal conditioner commercially available in Germany in April 2014 for 1 minute and then blow-dried.

It has been obtained a hair strand having a first and a second patterns. The hair fibers comprised the first pattern (where the adhesive composition has been applied) which had the initial blond (9/0) shade; and the second pattern comprised all the hair fibers without the first pattern. The second pattern had the red color shade (66/46).

Hence, when the adhesive composition comprised as the film-forming polymer Eastman AQ™ 38S Polymer, the adhesive composition is able to cover and temporary protect the first plurality of hair fiber portions from the hair color composition subsequently applied to all the hair fibers.

### Example 3

0.30 g of the adhesive composition Example 13 was applied to a first plurality of hair fiber portions of a 1.15 g of the above-identified blond (9/0) hair strand by using as an application tool to form a first pattern. The application tool was a bottle containing the adhesive composition and fitted with a relatively slim nozzle (see for instance Fig. 2). The first pattern was a plurality of regular and repetitive circular stripes. The hair strand was dried with a hair dryer for 20 min.

A hair coloring composition has been prepared by mixing a dye composition consisting of 10 g of Wella™ Koleston Perfect 66/46 (red color) and a developer composition consisting of 10 g of Wella™ Welloxon Perfect Developer Cream, 6% wt. hydrogen peroxide in 10 g of water.

The resulting hair coloring composition was applied to all the hair fibers of the dried hair strands. The hair strand was left to stand for 30 minutes at 25°C. After 30 minutes, the hair strand was rinsed for 2 min under tap water (6 L/min, 35°C) and then blow-dried.

After the adhesive composition and the hair coloring composition are rinsed off the hair for 2 minutes, the hair is then shampooed with Wella™ Professionals Brillance, Fine/Normal shampoo commercially available in Germany in April 2014 and conditioned with Wella™ Professionals Brillance, Fine/Normal conditioner commercially available in Germany in April 2014 for 1 minute and then blow-dried.

It has been obtained a hair strand having a first and a second patterns. The hair fibers comprised the first pattern (where the adhesive composition has been applied) which had the initial blond (9/0) shade; and the second pattern comprised all the hair fibers without the first pattern. The second pattern had the red color shade (66/46).

Hence, when the adhesive composition comprised as the film-forming polymer Fixate™ Plus Polymer, the adhesive composition is able to cover and temporary protect the first plurality of hair fiber portions from the hair color composition subsequently applied to all the hair fibers.

It has been also observed that the adhesive composition comprising as the film-forming polymer Fixate™ Plus Polymer and Eastman AQ™ 2350 Polymer could be readily washed off the hair compared the adhesive composition using Eastman AQ™ 38S Polymer as the film-forming polymer which has needed some additional time to be washed off from the hair fibers.

### EXPERIMENTAL

### Viscosity Test Method

The composition may have a kinematic viscosity of from 0,1 Pa.s to 75 Pa.s, measured at 25°C according to the following method. "Viscosity" can mean dynamic viscosity (measured in mPa·s) of a liquid at 25°C and ambient conditions. Dynamic viscosity may be measured using a rotational viscometer, such as a Brookfield Dial Reading Viscometer Model 1-2 RVT available from Brookfield Engineering Laboratories (USA) or other substitutable model as known in the art. Typical Brookfield spindles which may be used include, without limitation, LV-2 at a spindle speed of 20 rpm, recognizing that the exact spindle may be selected as needed by one skilled in the art. Kinematic viscosity may be determined by dividing dynamic viscosity by the density of the liquid (at 25°C and ambient conditions), as known in the art.

## Claims

1. A method for coloring hair fibers into a first and a second patterns, preferably human hair fibers, wherein the hair fibers have one or more initial color shades, the method for coloring the hair fibers comprising the following steps in that order:
(a) providing an adhesive composition, wherein the adhesive composition comprises, in a cosmetically acceptable carrier, a film-forming polymer in a total amount of film-forming polymer ranging from 10% to 75%, preferably from 15% to 70%, more preferably from 20% to 60%, even more preferably from 25% to 50% by total weight of the adhesive composition;
wherein the film-forming polymer is water-soluble or water-dispersible at 25°C; wherein the film-forming polymer has a glass transition temperature from -20°C to 100°C, preferably from -20°C to 70°C, more preferably from 0°C to 65°C;
wherein the film-forming polymer comprises one or more aryl-sulfonated polyester being a polycondensate of at least one dicarboxylic acid or of an ester thereof, of at least one diol and of at least one sulfoaryldicarboxylic difunctional compound substituted on the aromatic nucleus with a sulfonated group -SO₃M in which M represents a hydrogen atom, an ammonium ion, substituted ammonium or a metal ion such as Na⁺, Li⁺ or K⁺; and
wherein the aryl-sulfonated polyester is obtained from isophthalic acid, from the sodium salt of sulfoisophthalic acid, from diethylene glycol and from 1,4-cyclohexane-methanol ; or
wherein the film-forming polymer comprises one or more acrylic copolymers being cross-linked acrylic copolymers obtained by polymerization from a monomer composition comprising three different monomer components a1), b1, c1), and an optional fourth monomer component d1); wherein the first monomer component a1) is (meth)acrylic acid; wherein the second monomer component b1) is selected from at least one monoethylenically unsaturated monomer containing at least one linear or branched C1 to C4 alkyl ester of (meth)acrylic acid; wherein the third monomer component c1) is a mixture of crosslinking monomers selected from at least two different polyunsaturated monomer types or classes; and wherein the optional fourth monomer d1) is a monoethylenically unsaturated monomer different from monomer components a1), b1), and c1);
wherein the adhesive composition has a viscosity of from 0,1 Pa.s to 75 Pa.s, preferably from 0,4 Pa.s to 3 Pa.s according to the Viscosity Test Method as disclosed herein
(b) providing a hair coloring composition for forming a final color shade, which differ from the one or more initial color shades;
(c) applying the adhesive composition to a first plurality of hair fiber portions to form a first pattern, wherein the first plurality of hair fiber portions are covered and immobilized by the first pattern;
(d) drying the first plurality of hair fiber portions;
(e) applying the hair coloring composition to a second plurality of hair fiber portions including the first plurality of hair fiber portions comprising the first pattern, preferably to all the hair fibers;
(f) rinsing the adhesive composition and the hair coloring composition out of the hair fibers, and drying the hair fibers, to form a second pattern, wherein the second pattern comprises the second plurality of hair fiber portions without the first pattern, such that the hair fibers comprises the first pattern which has the one or more initial color shades and the second pattern which has the final color shade.

2. The method for coloring hair fibers into a first and a second patterns according to claim 1, wherein the adhesive composition is substantially free of organic solvents.

3. The method for coloring hair fibers into a first and a second patterns according to any preceding claims, wherein the adhesive composition is substantially free of surfactants, or comprises a total amount of surfactants ranging from 1% to 10%, preferably from 1.5% to 5%, by total weight of the adhesive composition.

4. The method for coloring hair into a first and a second patterns according to any preceding claims, wherein the hair coloring composition comprises a dye composition and optionally a developer composition wherein the developer composition comprises one or more oxidizing agents; and wherein the dye composition comprises one or more oxidative dye precursors comprising one or more couplers and one or more primary intermediates or wherein the dye composition consists essentially of one or more direct dyes.

5. The method for coloring hair fibers into a first and a second patterns according to Claim 4 wherein the one or more primary intermediates are selected from the group consisting of toluene-2,5-diamine, p-phenylenediamine, N-phenyl-p-phenylenediamine, N,N-bis(2-hydroxyethyl)-p-phenylenediamine, 2-hydroxyethyl-p-phenylenediamine, hydroxypropyl-bis-(N-hydroxyethyl-p-phenylenediamine), 2-methoxymethyl-p-phenylenediamine, 2-(1,2-dihydroxyethyl)-p-phenylenediamine, 2,2'-(2-(4-aminophenylamino)ethylazanediyl)diethanol, 2-(2,5-diamino-4-methoxyphenyl) propane-1,3-diol, 2-(7-amino-2H-benzo[b][1,4]oxazin-4(3H)-yl)ethanol, 2-chloro-p-phenylenediamine, p-aminophenol, p-(methylamino)phenol, 4-amino-m-cresol, 6-amino-m-cresol, 5-ethyl-o-aminophenol, 2-methoxy-p-phenylenediamine, 2,2'-methylenebis-4-aminophenol, 2,4,5,6-tetraminopyrimidine, 2,5,6-triamino-4-pyrimidinol, 1-hydroxyethyl-4,5-diaminopyrazole sulfate, 4,5-diamino-1-methylpyrazole, 4,5-diamino-1-ethylpyrazole, 4,5-diamino-1-isopropylpyrazole, 4,5-diamino-1-butylpyrazole, 4,5-diamino-1-pentylpyrazole, 4,5-diamino-1-benzylpyrazole, 2,3-diamino-6,7-dihydropyrazolo[1,2-a]pyrazol-1 (5H)-one dimethosulfonate, 4,5-diamino-1-hexylpyrazole, 4,5-diamino-1-heptylpyrazole, methoxymethyl-1,4-diaminobenzene, N,N-bis(2-hydroxyethyl)-N-(4-aminophenyl)-1,2-diaminothane, 2-[(3-aminopyrazolo[1,5-a]pyridin-2-yl]oxy]ethanol hydrochloride, salts thereof and mixtures thereof.

6. The method for coloring hair fibers into a first and a second patterns according to any of the Claims 4-5, wherein the dye composition of the hair coloring composition is substantially free of oxidizing agent.

7. The method for coloring hair fibers into a first and a second patterns according to any of the Claims 4-6, wherein the dye composition of the hair coloring composition further comprises beyond one or more one or more oxidative dye precursors, one or more direct dyes, preferably one or more oxidatively stable direct dyes.

8. The method for coloring hair fibers into a first and a second patterns according to any preceding claims further comprising a step of bleaching the hair between steps (d) and (e), wherein the step of bleaching is achieved by using one or more bleaching oxidizing agents selected from the group consisting of hydrogen peroxide, persulfate, perboric acid peracetic acid, their salts thereof, and mixtures thereof.

9. The method for coloring hair fibers into a first and a second patterns according to any preceding claims wherein the adhesive composition is applied to the first plurality of hair fiber portions by an application tool selected from the group consisting of a relatively slim nozzle applicator, a sponge brush applicator, a spatula, a relatively fine paint-brush applicator, and combinations thereof.

10. A kit for coloring hair fibers into a first and a second patterns, preferably human hair fibers, comprising:
(a) an adhesive composition,
wherein the adhesive composition comprises, in a cosmetically acceptable carrier, a film-forming polymer in a total amount of film-forming polymer ranging from 10% to 75%, preferably from 15% to 70%, more preferably from 20% to 60%, even more preferably from 25% to 50% by total weight of the adhesive composition;
wherein the film-forming polymer is water-soluble or water-dispersible at 25°C; wherein the film-forming polymer has a glass transition temperature from -20°C to 100°C, preferably from -20°C to 70°C, more preferably from 0°C to 65°C;
wherein the adhesive composition has a viscosity of from 0,1 Pa.s to 75 Pa.s, preferably from 0,4 Pa.s to 3 Pa.s according to the Viscosity Test Method as disclosed herein; and
wherein the film-forming polymer comprises one or more aryl-sulfonated polyester being a polycondensate of at least one dicarboxylic acid or of an ester thereof, of at least one diol and of at least one sulfoaryldicarboxylic difunctional compound substituted on the aromatic nucleus with a sulfonated group -SO₃M in which M represents a hydrogen atom, an ammonium ion, substituted ammonium or a metal ion such as Na⁺, Li⁺ or K⁺; and
wherein the aryl-sulfonated polyesters are obtained from isophthalic acid, from the sodium salt of sulfoisophthalic acid, from diethylene glycol and from 1,4-cyclohexane-methanol; or
wherein the film-forming polymer comprises one or more acrylic copolymers being cross-linked acrylic copolymers obtained by polymerization from a monomer composition comprising three different monomer components a1), b1, c1), and an optional fourth monomer component d1); wherein the first monomer component a1) is (meth)acrylic acid; wherein the second monomer component b1) is selected from at least one monoethylenically unsaturated monomer containing at least one linear or branched C₁ to C₄ alkyl ester of (meth)acrylic acid; wherein the third monomer component c1) is a mixture of crosslinking monomers selected from at least two different polyunsaturated monomer types or classes; and wherein the optional fourth monomer d1) is a monoethylenically unsaturated monomer different from monomer components a1), b1), and c1);
(b) a dye composition, preferably comprising one or more oxidative dye precursors or consisting essentially of one or more direct dyes;
(c) optionally, a developer composition comprising one or more oxidizing agents;
wherein the adhesive composition, the dye composition (b) and optionally the developer composition (c) are separately packaged in different containers or packaged in a same container in different compartments; and
(d) an instructional material for preparation, for use, or both preparation and use, of the adhesive composition, the dye composition and optionally the developer composition.

11. The kit for coloring hair fibers into a first and a second patterns of Claim 10, wherein the adhesive composition is supplied as a single-use disposable dose.

## Patentansprüche

1. Verfahren zum Färben von Haarfasern zu einem ersten und einem zweiten Muster, bevorzugt menschliche Haarfasern, wobei die Haarfasern einen oder mehrere Ausgangsfarbtöne aufweisen, wobei das Verfahren zum Färben der Haarfasern die folgenden Schritte in dieser Reihenfolge umfasst:
(a) Bereitstellen einer Klebstoffzusammensetzung, wobei die Klebstoffzusammensetzung in einem kosmetisch akzeptablen Träger ein filmbildendes Polymer in einer Gesamtmenge eines filmbildenden Polymers in einem Bereich von 10 % bis 75 %, vorzugsweise von 15 % bis 70 %, besonders bevorzugt von 20 % bis 60 %, ganz besonders bevorzugt von 25 % bis 50 %, bezogen auf das Gesamtgewicht der Klebstoffzusammensetzung, umfasst;
wobei das filmbildende Polymer bei 25 °C wasserlöslich oder wasserdispergierbar ist;
wobei das filmbildende Polymer eine Glasübergangstemperatur von - 20 °C bis 100 °C aufweist, bevorzugt von -20 °C bis 70 °C, besonders bevorzugt von 0 °C bis 65 °C;
wobei das filmbildende Polymer einen oder mehrere arylsulfonierte Polyester umfasst, die ein Polykondensat von zumindest einer Dicarbonsäure oder eines Esters davon, von zumindest einem Diol und von zumindest einer Sulfoaryldicarboxylic-difunktionellen Verbindung sind, die auf dem aromatischen Kern mit einer sulfonierten Gruppe -SO₃M substituiert ist, wobei M ein Wasserstoffatom, ein Ammoniumion, substituiertes Ammonium oder ein Metallion wie etwa Na⁺, Li⁺ oder K⁺ darstellt; und wobei der arylsulfonierte Polyester aus Isophthalsäure, aus dem Natriumsalz von Sulfoisophthalsäure, aus Diethylenglycol und aus 1,4-Cyclohexan-methanol gewonnen wird;
oder
wobei das filmbildende Polymer ein oder mehrere Acrylpolymere umfasst, die vernetzte Acrylcopolymere sind, die durch Polymerisierung aus einer Monomer-Zusammensetzung gewonnen werden, die drei verschiedene Monomerkomponenten a1), b1, c1) und eine optionale vierte Monomerkomponente d1) umfasst; wobei die erste Monomerkomponente a1) (Meth)acrylsäure ist; wobei die zweite Monomerkomponente b1) aus zumindest einem monoethylenisch ungesättigten Monomer ausgewählt wird, das zumindest einen linearen oder verzweigten C1 bis C4-Alkylester von (Meth)acrylsäure beinhaltet; wobei die dritte Monomerkomponente c1) ein Gemisch aus vernetzten Monomeren ist, die aus zumindest zwei verschiedenen mehrfach ungesättigten Monomertypen oder - klassen ausgewählt werden; und wobei das optionale vierte Monomer d1) ein monoethylenisch ungesättigtes Monomer ist, das von den Monomerkomponenten a1), b1) und c1) verschieden ist;
wobei die Klebstoffzusammensetzung eine Viskosität von 0,1 Pa*s bis 75 Pa*s aufweist, bevorzugt von 0,4 Pa*s bis 3 Pa*s
gemäß dem hierin offengelegten Viskositätsprüfungsverfahren aufweist
(b) Bereitstellen einer Haarfärbezusammensetzung zur Ausbildung eines endgültigen Farbtons, der sich von dem einen oder den mehreren Ausgangsfarbtönen unterscheidet;
(c) Auftragen der Klebstoffzusammensetzung auf eine erste Vielzahl von Haarfaserabschnitten, um ein erstes Muster zu bilden, wobei die erste Vielzahl von Haarfaserabschnitten durch das erste Muster abgedeckt und immobilisiert wird;
(d) Trocknen der ersten Vielzahl von Haarfaserabschnitten;
(e) Auftragen der Haarfärbezusammensetzung auf eine zweite Vielzahl von Haarfaserabschnitten, einschließlich der ersten Vielzahl von Haarfaserabschnitten, die das erste Muster umfasst, bevorzugt auf alle Haarfasern;
(f) Ausspülen der Klebstoffzusammensetzung und der Haarfärbezusammensetzung aus den Haarfasern und Trocknen der Haarfasern, um ein zweites Muster zu bilden, wobei das zweite Muster die zweite Vielzahl von Haarfaserabschnitten ohne das erste Muster umfasst, sodass die Haarfasern das erste Muster, das den einen oder die mehreren Ausgangsfarbtöne aufweist, und das zweite Muster, das den endgültigen Farbton aufweist, umfasst.

2. Verfahren zum Färben von Haarfasern zu einem ersten und einem zweiten Muster nach Anspruch 1, wobei die Klebstoffzusammensetzung im Wesentlichen frei von organischen Lösungsmitteln ist.

3. Verfahren zum Färben von Haarfasern zu einem ersten und einem zweiten Muster nach einem der vorhergehenden Ansprüche, wobei die Klebstoffzusammensetzung im Wesentlichen frei von Tensiden ist oder eine Gesamtmenge an Tensiden im Bereich von 1 % bis 10 %, bevorzugt von 1,5 % bis 5 %, bezogen auf das Gesamtgewicht der Klebstoffzusammensetzung, umfasst.

4. Verfahren zum Färben von Haaren zu einem ersten und einem zweiten Muster nach einem der vorhergehenden Ansprüche, wobei die Haarfärbezusammensetzung eine Farbstoffzusammensetzung und optional eine Entwicklerzusammensetzung umfasst, wobei die Entwicklerzusammensetzung ein oder mehrere Oxidationsmittel umfasst; und wobei die Farbstoffzusammensetzung ein oder mehrere Oxidationsfarbstoffvorprodukte umfasst, die einen oder mehrere Haftvermittler und ein oder mehrere primäre Zwischenprodukte umfassen, oder wobei die Farbstoffzusammensetzung im Wesentlichen aus einem oder mehreren Direktfarbstoffen besteht.

5. Verfahren zum Färben von Haarfasern zu einem ersten und einem zweiten Muster gemäß
Anspruch 4, worin das eine oder die mehreren primären Zwischenprodukte aus der Gruppe ausgewählt werden, die aus Toluol-2,5-diamin, p-Phenylendiamin, N-Phenyl-p-phenylendiamin, N,N-bis(2-Hydroxethyl)-p-phenylendiamin, 2-Hydroxyethyl-p-phenylendiamin, Hydroxypropyl-bis-(N-hydroxyethyl-p-phenylendiamin),2-Methoxymethyl-p-phenylendiamin, 2-(1,2-Dihydroxyethyl)-p-phenylendiamin, 2,2'-(2-(4-Aminophenylamino)ethylazandiyl)diethanol, 2-(2,5-Diamino-4-methoxyphenyl)propan-1,3-diol,2-(7-Amino-2H-benzo[b] [1,4]oxazin-4(3H)-yl)ethanol, 2-Chloro-p-phenylendiamin, p-Aminophenol, p-(Methylamino)phenol, 4-Amino-m-kresol, 6-Amino-m-kresol, 5-Ethyl-o-aminophenol, 2-Methoxy-p-phenylendiamin, 2,2'-Methylenbis-4-aminophenol, 2,4,5,6-Tetraminopyrimidin, 2,5,6-Triamino-4-pyrimidinol, 1-Hydroxyethyl-4,5-diaminopyrazolsulfat, 4,5-Diamino-1-methylpyrazol, 4,5-Diamino-1-ethylpyrazol, 4,5-Diamino-1-isopropylpyrazol, 4,5-Diamno-1-butylpyrazol, 4,5-Diamino-l-pentylpyrazol, 4,5-Diamino-1-benzylpyrazol, 2,3-Diamino-6,7-dihydropyrazolo[1,2-a]pyrazol-1-(5H)-on-dimethosulfonat, 4,5-Diamino-1-hexylpyrazol, 4,5-Diamino-1-heptylpyrazol, Methoxymethyl-1,4-diaminobenzen, N,N-bis(2-Hydroxyethyl)-N-(4-aminophenyl)-1,2-diaminoethan, 2-[(3-Aminopyrazolo[1,5-a]pyridin-2-yl)oxy]ethanolhydrochlorid, Salze davon und Mischungen davon umfasst.

6. Verfahren zum Färben von Haarfasern zu einem ersten und einem zweiten Muster nach einem der Ansprüche 4-5, wobei die Farbstoffzusammensetzung der Haarfärbezusammensetzung im Wesentlichen frei von Oxidationsmitteln ist,

7. Verfahren zum Färben von Haarfasern zu einem ersten und einem zweiten Muster nach einem der Ansprüche 4-6, wobei die Farbstoffzusammensetzung der Haarfärbezusammensetzung ferner zusätzlich zu einem oder mehreren Oxidationsfarbstoffvorprodukten, einen oder mehrere Direktfarbstoffe umfasst, bevorzugt einen oder mehrere oxidativ stabile Direktfarbstoffe.

8. Verfahren zum Färben von Haarfasern zu einem ersten und einem zweiten Muster nach einem der vorhergehenden Ansprüche, ferner einen Schritt des Bleichens der Haare zwischen den Schritten (d) und (e) umfassend, wobei der Schritt des Bleichens durch Verwenden eines oder mehrerer Bleichoxidationsmittel erreicht wird, die aus der Gruppe ausgewählt werden, die aus Wasserstoffperoxid, Persulfat, Perborsäure, Peressigsäure, Salzen davon und Mischungen davon besteht.

9. Verfahren zum Färben von Haarfasern zu einem ersten und einem zweiten Muster nach einem der vorhergehenden Ansprüche, wobei die Klebstoffzusammensetzung durch ein Aufbringungswerkzeug, das aus der Gruppe ausgewählt wird, die aus einem relativ dünnen Düsenapplikator, einem Schwammbürstenapplikator, einem Spatel, einem relativ feinen Pinselapplikator und Kombinationen davon besteht, auf die erste Vielzahl von Haarfaserabschnitten aufgebracht wird.

10. Kit zum Färben von Haarfasern zu einem ersten und einem zweiten Muster, bevorzugt menschliche Haarfasern, umfassend:
(a) eine Klebstoffzusammensetzung,
wobei die Klebstoffzusammensetzung in einem kosmetisch akzeptablen Träger ein filmbildendes Polymer in einer Gesamtmenge eines filmbildenden Polymers in einem Bereich von 10 % bis 75 %, vorzugsweise von 15 % bis 70 %, besonders bevorzugt von 20 % bis 60 %, ganz besonders bevorzugt von 25 % bis 50 %, bezogen auf das Gesamtgewicht der Klebstoffzusammensetzung, umfasst;
wobei das filmbildende Polymer bei 25 °C wasserlöslich oder wasserdispergierbar ist; wobei das filmbildende Polymer eine Glasübergangstemperatur von -20 °C bis 100 °C aufweist, bevorzugt von -20 °C bis 70 °C, besonders bevorzugt von 0 °C bis 65 °C;
wobei die Klebstoffzusammensetzung eine Viskosität von 0,1 Pa*s bis 75 Pa*s aufweist, bevorzugt von 0,4 Pa*s bis 3 Pa*s, gemäß dem hierin offengelegten Viskositätsprüfungsverfahren; und
wobei das filmbildende Polymer einen oder mehrere arylsulfonierte Polyester umfasst, die ein Polykondensat von zumindest einer Dicarbonsäure oder eines Esters davon, von zumindest einem Diol und von zumindest einer Sulfoaryldicarboxylic-difunktionellen Verbindung sind, die auf dem aromatischen Kern mit einer sulfonierten Gruppe -SO₃M substituiert ist, wobei M ein Wasserstoffatom, ein Ammoniumion, substituiertes Ammonium oder ein Metallion wie etwa Na⁺, Li⁺ oder K⁺ darstellt; und wobei die arylsulfonierten Polyester aus Isophthalsäure, aus dem Natriumsalz von Sulfoisophthalsäure, aus Diethylenglycol und aus 1,4-Cyclohexan-methanol gewonnen werden; oder
wobei das filmbildende Polymer ein oder mehrere Acrylpolymere umfasst, die vernetzte Acrylcopolymere sind, die durch Polymerisierung aus einer Monomer-Zusammensetzung gewonnen werden, die drei verschiedene Monomerkomponenten a1), b1, c1) und eine optionale vierte Monomerkomponente d1) umfasst; wobei die erste Monomerkomponente a1) (Meth)acrylsäure ist; wobei die zweite Monomerkomponente b1) aus zumindest einem monoethylenisch ungesättigten Monomer ausgewählt wird, das zumindest einen linearen oder verzweigten C₁ bis C₄-Alkylester von (Meth)acrylsäure beinhaltet; wobei die dritte Monomerkomponente c1) ein Gemisch aus vernetzten Monomeren ist, die aus zumindest zwei verschiedenen mehrfach ungesättigten Monomertypen oder -klassen ausgewählt werden; und wobei das optionale vierte Monomer d1) ein monoethylenisch ungesättigtes Monomer ist, das von den Monomerkomponenten a1), b1) und c1) verschieden ist;
(b) Farbstoffzusammensetzung, bevorzugt ein oder mehrere Oxidationsfarbstoffvorprodukte umfassend oder im Wesentlichen aus einem oder mehreren Direktfarbstoffen bestehend;
(c) optional eine Entwicklerzusammensetzung, die ein oder mehrere Oxidationsmittel umfasst, wobei die Klebstoffzusammensetzung, die Farbstoffzusammensetzung (b) und optional die Entwicklerzusammensetzung (c) getrennt in verschiedenen Behältern oder in einem einzelnen Behälter in verschiedenen Kammern verpackt sind; und
(d) eine Anleitung zur Herstellung, Verwendung oder sowohl Herstellung als auch Verwendung der Klebstoffzusammensetzung, der Farbstoffzusammensetzung und optional der Entwicklerzusammensetzung.

11. Kit zum Färben von Haarfasern zu einem ersten und einem zweiten Muster nach Anspruch 10, wobei die Klebstoffzusammensetzung als Einweg-Wegwerfdosis bereitgestellt wird.

## Revendications

1. Procédé pour la coloration de fibres capillaires dans un premier et un deuxième motif, de préférence des fibres capillaires humaines, dans lequel les fibres capillaires ont une ou plusieurs nuances initiales de couleur, le procédé pour la coloration des fibres capillaires comprenant les étapes suivantes dans cet ordre :
(a) la fourniture d'une composition adhésive, dans laquelle la composition adhésive comprend, dans un support cosmétiquement acceptable, un polymère formant un film dans une quantité totale de polymère formant des films allant de 10% à 75%, de préférence de 15% à 70%, plus de préférence de 20% à 60%, encore plus de préférence de 25% à 50% sur le poids total de la composition adhésive ;
dans lequel le polymère formant un film est soluble dans l'eau ou dispersible dans l'eau à 25 °C ;
dans lequel le polymère formant un film a une température de transition vitreuse de -20°C à 100°C, de préférence de -20°C à 70°C, plus de préférence de 0°C à 65°C ;
dans lequel le polymère formant un film comprend un ou plusieurs polyester arylsulfoné étant un polycondensat d'au moins un acide dicarboxylique ou d'un de ses esters, d'au moins un diol et d'au moins un composé difonctionnel sulfoaryldicarboxylique substitué sur le noyau aromatique par un groupe sulfoné -SO₃M dans lequel M représente un atome d'hydrogène, un ion d'ammonium, un ammonium substitué ou un ion métallique tel que Na⁺, Li⁺ or K⁺, et dans lequel le polyester arylsulfoné est obtenu à partir d'acide isophtalique, à partir du sel de sodium de l'acide sulfoisophtalique, à partir de diéthylène glycol et de 1,4-cyclohexane-méthanol;
ou
dans lequel le polymère formant un film comprend un ou plusieurs copolymères acryliques étant des copolymères acryliques réticulés obtenus par polymérisation à partir d'une composition monomère comprenant trois composants monomères différents a1), b1, c1), et un quatrième composant monomère facultatif d1) ; dans lequel le premier composant monomère a1) est l'acide (méth)acrylique ; dans lequel le deuxième composant monomère b1) est choisi parmi au moins un monomère monoéthyléniquement insaturé comprenant au moins un ester alkylique linéaire ou ramifié C1 à C4 d'acide (méth)acrylique ; dans lequel le troisième composant monomère c1) est un mélange de monomères réticulants choisis parmi au moins deux types ou classes de monomères polyinsaturés différents ; et
dans lequel le quatrième monomère facultatif d1) est un monomère monoéthyléniquement insaturé différent des composants monomères a1), b1) et c1) ;
dans lequel la composition adhésive a une viscosité de 0,1 Pa.s à 75 Pa.s, de préférence de 0,4 Pa.s à 3 Pa.s conformément à la méthode de test de viscosité telle que décrite dans les présentes
(b) la fourniture d'une composition de coloration capillaire pour la formation d'une teinte finale de couleur, qui diffère de la ou des plusieurs nuances initiales de couleur ;
(c) l'application de la composition adhésive à une première pluralité de parties de fibres capillaires pour former un premier motif, dans lequel la première pluralité des parties de fibres capillaires est couverte et immobilisée par le premier motif ;
(d) le séchage de la première pluralité de parties de fibres capillaires ;
(e) l'application de la composition de coloration capillaire à une deuxième pluralité de parties de fibres capillaires, y compris la première pluralité de parties de fibres capillaires comprenant le premier motif, de préférence à toutes les fibres capillaires ;
(f) le rinçage de la composition adhésive et de la composition de coloration capillaire hors des fibres capillaires, et le séchage des fibres capillaires, pour former un deuxième motif, dans lequel le deuxième motif comprend la deuxième pluralité de parties de fibres capillaires sans le premier motif, de sorte que les fibres capillaires comprennent le premier motif qui a le ou les nuances de couleur initiales et le deuxième motif qui a la teinte finale de couleur.

2. Procédé pour la coloration de fibres capillaires dans un premier et un deuxième motif selon la revendication 1, dans lequel la composition adhésive est sensiblement exempte de solvants organiques.

3. Procédé pour la coloration des fibres capillaires dans un premier et un deuxième motif selon l'une quelconque des revendications précédentes, dans lequel la composition adhésive est sensiblement exempte de surfactants, ou comprend une quantité totale de surfactants allant de 1% à 10%, de préférence de 1,5% à 5%, sur le poids total de la composition adhésive.

4. Procédé pour la coloration des cheveux dans un premier et un deuxième motif selon l'une quelconque des revendications précédentes, dans lequel la composition de coloration capillaire comprend une composition de colorant et éventuellement une composition de développeur dans laquelle la composition de développeur comprend un ou plusieurs agents oxydants et dans lequel la composition de colorant comprend un ou plusieurs précurseurs de colorant oxydatif comprenant un ou plusieurs coupleurs et un ou plusieurs intermédiaires primaires ou dans lequel la composition de colorant comprend essentiellement un ou plusieurs colorants directs,

5. Procédé pour la coloration de fibres capillaires dans un premier et un deuxième motif selon la
Revendication 4, dans lequel l'un ou plusieurs intermédiaires primaires sont sélectionnés parmi le groupe constitué de toluène-2,5-diamine, p-phénylénédiamine, N-phényl-p-phénylénédiamine, N,N-bis(2-hydroxyéthyl)-p-phénylénédiamine, 2-hydroxyéthyl-p-phénylénédiamine, hydroxypropyl-bis-(N-hydroxyéthyl-p-phénylénédiamine), 2-méthoxyméthyl-p-phénylénédiamine, 2-(1,2-dihydroxyéthyl}-p-phénylenediamine, 2,2'-(2-(4-aminophénylarnino)éthyl-zanédiyl)diéthanol, 2-(2,5-diamino-4-méthoxyphényl)propane-1,3-diol, 2-(7-amino-2H-benzo[b][1,4]oxazin-4(3H)-yl)éthanol, 2-chloro-p-phénylènediamine, p-aminophénol, p-(méthylamino)phénol, 4-amino-m-crésol, 6-amino-m-crésol, 5-éthyl-o-aminophénol, 2-méthoxy-p-phénylènediamine, 2,2'-méthylénebis-4-aminophénol, 2,4,5,6-tétraminopyrimidine, 2,5,6-triamino-4-pyrimidinol, sulfate de 1-hydroxyéthyl-4,5-diaminopyrazole, 4,5-diamino-1-méthylpyrazole, 4,5-diamino-1-éthylpyrazole, 4,5-diamino-1-isopropylpyrazole, 4,5-diamino-1-butylpyrazole, 4,5-diamino-1-pentylpyrazole, 4, Diméthosulfonate de 5-diamino-1-benzylpyrazole, 2,3-diamino-6,7-dihydropyrazolo[1,2-a] pyrazole-1(5H)-one, 4,5-diamino-1-hexylpyrazole, 4,5-diamino-1-heptylpyrazole, méthoxyméthyl-1,4-diaminobenzène, N,N-bis(2-hydroxyéthyl)-N-(4-aminophényl)-1,2-diaminothane, 2-[(3-aminopyrazolo[1,Chlorhydrate de 5-a] pyridin-2-yl)oxy]éthanol, leurs sels et leurs mélanges.

6. Procédé pour la coloration de fibres capillaires dans un premier et un deuxième motif selon l'une quelconque des revendications 4-5, dans lequel la composition de colorant de la composition de coloration capillaire est sensiblement exempte d'agent oxydant,

7. Procédé pour la coloration des fibres capillaires dans un premier et un deuxième motif selon l'une quelconque des revendications 4-6, dans lequel la composition de colorant de la composition de coloration capillaire comprend en outre au-delà d'un ou plusieurs un ou plusieurs précurseurs de colorants oxydants, un ou plusieurs colorants directs, de préférence un ou plusieurs colorants directs stables à l'oxydation.

8. Procédé pour la coloration de fibres capillaires dans un premier et un deuxième motif selon l'une quelconque des revendications précédentes, comprenant en outre une étape de décoloration des cheveux entre les étapes (d) et (e), dans lequel l'étape de décoloration est obtenue à l'aide d'un ou plusieurs agents oxydants de décoloration sélectionnés dans le groupe constitué de peroxyde d'hydrogène, de persulfate, d'acide perborique, d'acide peracétique, de leurs sels et de leurs mélanges,

9. Procédé pour la coloration des fibres capillaires dans un premier et un deuxième motif selon l'une quelconque des revendications précédentes, dans lequel la composition adhésive est appliquée à la première pluralité de parties de fibres capillaires par un outil d'application sélectionné dans le groupe constitué d'un applicateur à buse relativement mince, d'une éponge applicateur de pinceau, d'une spatule, d'un applicateur de pinceau relativement fin et de leurs combinaisons,

10. Kit pour la coloration de fibres capillaires dans un premier et un deuxième motif, de préférence des fibres de cheveux humains, comprenant :
(a) une composition adhésive,
dans laquelle la composition adhésive comprend, dans un support cosmétiquement acceptable, un polymère formant un film dans une quantité totale de polymère formant des films allant de 10% à 75%, de préférence de 15% à 70%, plus de préférence de 20% à 60%, encore plus de préférence de 25% à 50% sur le poids total de la composition adhésive ;
dans lequel le polymère formant un film est soluble dans l'eau ou dispersible à 25 °C, dans lequel le polymère formant un film a une température de transition vitreuse de -20°C à 100°C, de préférence de -20°C à 70°C, plus de préférence de 0°C à 65°C ;
dans lequel la composition adhésive a une viscosité de 0,1 Pa.s à 75 Pa.s, de préférence de 0,4 Pa.s à 3 Pa, conformément à la méthode de test de viscosité telle que décrite dans les présentes ; et
dans lequel le polymère formant un film comprend un ou plusieurs polyester arylsulfoné étant un polycondensat d'au moins un acide dicarboxylique ou d'un de ses esters, d'au moins un diol et d'au moins un composé difonctionnel sulfoaryldicarboxylique substitué sur le noyau aromatique par un groupe sulfoné -SO₃M dans lequel M représente un atome d'hydrogène, un ion d'ammonium, un ammonium substitué ou un ion métallique tel que Na⁺, Li⁺ or K⁺, et dans lequel les polyesters arylsulfonés sont obtenus à partir d'acide isophtalique, à partir du sel de sodium de l'acide sulfoisophtalique, à partir de diéthylène glycol et de 1,4-cyclohexane-méthanol ; ou
dans lequel le polymère formant un film comprend un ou plusieurs copolymères acryliques étant des copolymères acryliques réticulés obtenus par polymérisation à partir d'une composition monomère comprenant trois composants monomères différents a1), b1, c1), et un quatrième composant monomère facultatif d1) ; dans lequel le premier composant monomère a1) est l'acide (méth)acrylique ; dans lequel le deuxième composant monomère b1) est choisi parmi au moins un monomère monoéthyléniquement insaturé comprenant au moins un ester alkylique linéaire ou ramifié C₁ à C₄ d'acide (méth)acrylique ; dans lequel le troisième composant monomère c1) est un mélange de monomères réticulants choisis parmi au moins deux types ou classes de monomères polyinsaturés différents ; et dans lequel le quatrième monomère facultatif d1) est un monomère monoéthyléniquement insaturé différent des composants monomères a1), b1) et c1) ;
(b) une composition de colorant, comprenant de préférence un ou plusieurs précurseurs oxydatifs ou composé essentiellement d'un ou de plusieurs colorants directs ;
(c) éventuellement, une composition de révélateur comprenant un ou plusieurs agents oxydants, dans lequel la composition adhésive, la composition de colorant (b) et éventuellement la composition de révélateur (c) sont conditionnées séparément dans différents récipients ou conditionnées dans un même récipient dans différents compartiments ; et
(d) un matériau d'instructions destiné à la préparation, à l'utilisation, ou à la fois à la préparation et à l'utilisation, de la composition adhésive, de la composition de colorant et éventuellement de la composition de révélateur,

11. Kit pour la coloration des fibres capillaires dans un premier et un deuxième motif selon la revendication 10, dans lequel la composition adhésive est fournie comme dose jetable à usage unique.
